# EUROPEAN PATENT SPECIFICATION

(11) **EP 2 450 046 B1**
(45) Date of publication and mention of the grant of the patent: **24.06.2015**
(21) Application number: 09846679.0
(22) Date of filing: 30.06.2009
(51) Int. Cl.: A61K 36/8888, A61K 36/78, A61K 36/752, A61K 36/736, A61K 36/634, A61K 36/54, A61K 36/539, A61K 36/535, A61K 36/484, A61K 36/31, A61K 36/284, A61K 36/28, A61K 36/076, A61P 11/06, A61P 11/08, A61K 36/73, A61K 36/888, A61K 36/185

(54) **A MEDICINAL COMPOSITION FOR THE TREATMENT OF BRONCHITIS AND PREPARATION THEREOF**
MEDIZINISCHE ZUSAMMENSETZUNG ZUR BEHANDLUNG VON BRONCHITIS UND IHRE HERSTELLUNG
COMPOSITION À VISÉE MÉDICALE POUR LE TRAITEMENT DE LA BRONCHITE ET SA PRÉPARATION

(43) Date of publication of application: 09.05.2012
(73) Proprietor: Hebei Yiling Medicine Research Institute Co., Ltd., Hebei 050035 (CN)
(72) Inventor: WU, Yiling, Shijiazhuang Hebei 050035 (CN); ZHANG, Yongfeng, Shijiazhuang Hebei 050035 (CN); XU, Honghui, Shijiazhuang Hebei 050035 (CN); LI, Xiaoyan, Shijiazhuang Hebei 050035 (CN); JI, Xueli, Shijiazhuang Hebei 050035 (CN); WU, Xiaoli, Hebei 050035 (CN); WANG, Chao, Shijiazhuang Hebei 050035 (CN); LI, Yunpeng, Shijiazhuang Hebei 050035 (CN); WANG, Meng, Shijiazhuang Hebei 050035 (CN)
(74) Representative: Kador & Partner
(86) International application number: PCT/CN2009/072537
(87) International publication number: WO 2011/000150

(56) References cited:
- CN-A- 1 562 296
- CN-A- 1 593 560
- CN-A- 101 549 061
- ZHU YING; LIU XIAODONG: "Treatment of chronic bronchitis with modified ma xing shi gan tang and er chen tang.", JOURNAL OF TRADITIONAL CHINESE MEDICINE, vol. 24, no. 1, March 2004 (2004-03), pages 12-13, XP9167376,
- "Blue Poppy Press Recent Research Report 199: Cough", Blue Poppy Press Blue Poppy Press, 1995, pages 1-2, XP002692750, Retrieved from the Internet: URL:http://bluepoppy.com/cfwebstore/index. cfm/feature/477/research-report-199-cough. cfm [retrieved on 2013-02-25]
- DI B ET AL: "Pharmacokinetic comparisons of Shuang-Huang-Lian with the different combinations of its constitutional herbs", JOURNAL OF ETHNOPHARMACOLOGY, ELSEVIER SCIENTIFIC PUBLISHERS LTD, IE, vol. 107, no. 3, 11 October 2006 (2006-10-11), pages 401-405, XP027939592, ISSN: 0378-8741 [retrieved on 2006-10-11]
- DATABASE WPI Week 200482 Thomson Scientific, London, GB; AN 2004-822477 XP002692751, -& CN 1 528 391 A (ZHENGDA TIANQING PHARM CO LTD JIANGSU) 15 September 2004 (2004-09-15)
- DATABASE WPI Week 200239 Thomson Scientific, London, GB; AN 2002-353044 XP002692752, -& CN 1 336 214 A (CAO H) 20 February 2002 (2002-02-20)
- DATABASE WPI Week 200732 Thomson Scientific, London, GB; AN 2007-330115 XP002692753, -& CN 1 840 118 A (ZHANG Y) 4 October 2006 (2006-10-04)
- DATABASE WPI Week 200945 Thomson Scientific, London, GB; AN 2009-K84177 XP002692754, -& CN 101 455 822 A (LIU M) 17 June 2009 (2009-06-17)
- DATABASE WPI Week 200867 Thomson Scientific, London, GB; AN 2008-L32377 XP002692755, & CN 101 129 573 A (YIN K) 27 February 2008 (2008-02-27)
- DATABASE WPI Week 200525 Thomson Scientific, London, GB; AN 2005-234111 XP002692756, -& CN 1 557 432 A (SICHUAN RENDE PHARM CO LTD) 29 December 2004 (2004-12-29)
- DATABASE WPI Week 200627 Thomson Scientific, London, GB; AN 2006-254478 XP002692757, -& CN 1 679 864 A (ZHANG Y) 12 October 2005 (2005-10-12)
- DATABASE WPI Week 200632 Thomson Scientific, London, GB; AN 2006-300453 XP002692758, -& CN 1 706 467 A (GONG M) 14 December 2005 (2005-12-14)
- Anon: "Gui Long Ke Chuan Ning Capsule-For Wind Cold Cough", Herbsbuy300.com , 22 November 2011 (2011-11-22), XP002692759, Retrieved from the Internet: URL:http://www.herbsbuy360.com/gui-long-ke -chuan-ning-capsulefor-wind-cold-cough-p-1 94.html#.USc7LaywV7k [retrieved on 2013-02-25]

## Description

### Technical Field of the Invention

The invention belongs to the field of Traditional Chinese medicine, in particular to a pharmaceutical composition for treatment of bronchitis and preparation methods thereof.

### Background of the Invention

Bronchitis is an inflammation in the mucous membrane of trachea and bronchi due to bacterial and viral infections, or stimulations by physical and chemical factors. Typically, it is mainly characterized by cough, expectoration, substernal malaise or pain, panting, and the accompanying common symptoms of the cold. According to the period lasted, bronchitises can be classified into the two categories: the acute tracheobronchitis and the chronic bronchitis. The chronic bronchitis is a bronchitis that generally lasts for more than two months and attacks in two consecutive years, or lasts for three consecutive months within one year, and that causes inflammation on mucous membrane and peripheral tissues thereof. Most patients are adults, and are often attacked in spring and winter. The bronchitis belongs to the categories of the "cough", "phlegm-fluid retention", "asthma syndrome", etc, in the traditional Chinese medicine.

Chronic bronchitis refers to chronic, non-specific inflammation on the mucosa of trachea and bronchi as well as the peripheral tissues thereof, and characterizes clinically in cough, expectoration or a chronic course accompanied with panting and repeated onset. If the disease develops slowly, it is often complicated by obstructive pulmonary emphysema, even pulmonary hypertension or pulmonary heart disease. According to the statistics of partial general investigation in 1973 in China, the prevalence of chronic bronchitis was about 3.82%, and increased with age and was up to about 15% in the persons of above 50 years old. It is indicated from the statistics of partial general investigation in 1992 in China that the prevalence of chronic bronchitis was 3.2%.

The cause for chronic bronchitis is considerably complex. In addition to factors such as environment, climate, genetics, smoking, etc., viral and bacterial infections are extremely important factors. In addition to the above etiological factors, the body's internal factors are involved in chronic bronchitis, such as dysfunction of autonomic nerves that may lead to hyperfunctioning of parasympathetic nerves, such that the response of airway of such personis more sensitive than ordinary ones. Therefore, a weak stimulus that has no effect on ordinary persons may cause contract and spasm of bronchi, increase of secretion, and develop symptoms such as cough, expectoration, panting, etc. At present, treatment based on the western medicine mainly focuses on controling infection, dispelling phlegm and checking cought, while it is not effective in adjusting dysfunction of the internal factors. After enduring the acute phase for several days, many patients often experience relieving of pyrexia, aversion to cold, and sticky yellow phlegm. However, symptoms such as cough, expectoration and panting are not alleviated, and the sputum volume is significantly increased. Additionally, the long-term and massive application of the broad-spectrum antibiotics in combination with glucocorticoids is also a further cause for nosocomial secondary infection. There are investigations show that a treatment of chronic bronchitis with traditional Chinese medicine in appropriate proportions at different stages is optimal in respect to efficacy and economy.

The treatment of the disease has been a quit long history in traditional Chinese medicine. Continuous exploration has been lasting over generations of physicians on principles, methods, prescriptions and drugs for treatment of cough by traditional Chinese medicine. A treatment of traditional Chinese medicine emphasizes the principle of treating the incidental and fundamental simultaneously. Syndrome differentiation and treatment are performed at different stages which may be classified according to the development of the disease to such as the stage of exopathogen attacking the lung, the stage of turbid phlegm obstructing the lung, the stage of *qi* stagnation and blood stasis, and the stage of domination of pathogen over vital-*qi*. It has been shown by pharmacological investigations that the treatment of the chronic bronchitis with the traditional Chinese medicine embodies various effects, such as checking cough, resolving phlegm, relieving dyspnea, and anti-inflammation. It has been demonstrated in the clinical investigation that the treatment with traditional Chinese medicine can significantly relieve symptoms such as cough, expectoration, dyspnea and the like, while has fewer adverse reactions in comparison with the western medicine. Since most of patients can enter into the remission stage of chronic bronchitis during the treatment, frequency of application of antibiotics may be reduced, so that the patients' constitution can be strengthened, and intervals between acute onsets can be prolonged, and thereby the patient's quality of life can be improved.

Therefore, there is still a need in developing novel traditional Chinese medicine for treating the bronchitis, particularly the chronic bronchitis.

### Summary of the Invention

To meet the above-described need, the present invention provides a pharmaceutical composition for treating bronchitis and the preparation methods for the same. The pharmaceutical composition of the present invention is made from the medicinal materials in the following parts by weight:

| CITRI RETICULATAE | PINELLIAE RHIZOMA | ASTERIS RADIX ET | FARFARAE FLOS |
|---|---|---|---|
| PERICARPIUM 94 - 156 | 94 - 156 | RHIZOMA 94 - 156 | 94 - 156 |
| PORIA 94 - 156 | ATRACTYLODIS MACROCEPHALAE RHIZOMA 94-156 | ARMENIACAE SEMEN AMARUM 78 - 130 | PERILLAE FRUCTUS 78 - 130 |
| SINAPIS SEMEN 78-130 | RAPHANI SEMEN 78 - 130 | CINNAMOMI RAMULUS 46 - 78 | SCUTELLARIAE RADIX 94 - 156 |
| FORSYTHIAE FRUCTUS 94 - 156 | HOUTTUYNIAE HERBA 225 - 391 | and GLYCYRRHIZAE RADIX ET RHIZOMA 31 - 53. | |

| | | | |
|---|---|---|---|
| (Note: the above Latin names of the medicinal materials are according to Chinese Pharmacopoeia, Jan., 2010.) | | | |

Preferably, the pharmaceutical composition of the present invention is made from the medicinal materials in the following parts by weight:

| | | | |
|---|---|---|---|
| CITRI RETICULATAE PERICARPIUM 94 | PINELLIAE RHIZOMA 156 | ASTERIS RADIX ET RHIZOMA 94 | FARFARAE FLOS 156 |
| PORIA 94 | ATRACTYLODIS MACROCEPHALAE RHIZOMA 156 | ARMENIACAE SEMEN AMARUM 78 | PERILLAE FRUCTUS 130 |
| SINAPIS SEMEN 78 | RAPHANI SEMEN 130 | CINNAMOMI RAMULUS 46 | SCUTELLARIAE RADIX 94 |
| FORSYTHIAE FRUCTUS 156 | HOUTTUYNIAE HERBA 391 | and GLYCYRRHIZAE RADIX ET RHIZOMA 31. | |

| More preferably, the pharmaceutical composition of the present invention is made from the medicinal materials in the following parts by weight: | | | |
|---|---|---|---|
| CITRI RETICULATAE PERICARPIUM 156 | PINELLIAE RHIZOMA 94 | ASTERIS RADIX ET RHIZOMA 156 | FARFARAE FLOS 94 |
| PORIA 156 | ATRACTYLODIS MACROCEPHALAE RHIZOMA 94 | ARMENIACAE SEMEN AMARUM 130 | PERILLAE FRUCTUS 78 |
| SINAPIS SEMEN 130 | RAPHANI SEMEN 78 | CINNAMOMI RAMULUS 78 | SCUTELLARIAE RADIX 156 |
| FORSYTHIAE FRUCTUS 94 | HOUTTUYNIAE HERBA 225 | and GLYCYRRHIZAE RADIX ET RHIZOMA 53. | |

| Alternatively, the composition can be made from the medicinal materials in the following parts by weight: | | | |
|---|---|---|---|
| CITRI RETICULATAE PERICARPIUM 125 | PINELLIAE RHIZOMA 125 | ASTERIS RADIX ET RHIZOMA 125 | FARFARAE FLOS 125 |
| PORIA 125 | ATRACTYLODIS MACROCEPHALAE RHIZOMA 125 | ARMENIACAE SEMEN AMARUM 104 | PERILLAE FRUCTUS 104 |
| SINAPIS SEMEN 104 | RAPHANI SEMEN 104 | CINNAMOMI RAMULUS 62 | SCUTELLARIAE RADIX 125 |
| FORSYTHIAE FRUCTUS | HOUTTUYNIAE HERBA | and GLYCYRRHIZAE | |
| 125 | 313 | RADIX ET RHIZOMA 42. | |

| Alternatively, the composition can be made from the medicinal materials in the following parts by weight: | | | |
|---|---|---|---|
| CITRI RETICULATAE PERICARPIUM 98 | PINELLIAE RHIZOMA 98 | ASTERIS RADIX ET RHIZOMA 104 | FARFARAE FLOS 104 |
| PORIA 144 | ATRACTYLODIS MACROCEPHALAE RHIZOMA 148 | ARMENIACAE SEMEN AMARUM 85 | PERILLAE FRUCTUS 85 |
| SINAPIS SEMEN 125 | RAPHANI SEMEN 128 | CINNAMOMI RAMULUS 75 | SCUTELLARIAE RADIX 151 |
| FORSYTHIAE FRUCTUS 149 | HOUTTUYNIAE HERBA 377 | and GLYCYRRHIZAE RADIX ET RHIZOMA 49. | |

| Alternatively, the composition can be made from the medicinal materials in the following parts by weight: | | | |
|---|---|---|---|
| CITRI RETICULATAE PERICARPIUM 147 | PINELLIAE RHIZOMA 152 | ASTERIS RADIX ET RHIZOMA 149 | FARFARAE FLOS 140 |
| PORIA 105 | ATRACTYLODIS MACROCEPHALAE RHIZOMA 105 | ARMENIACAE SEMEN AMARUM 81 | PERILLAE FRUCTUS 81 |
| SINAPIS SEMEN 125 | RAPHANI SEMEN 125 | CINNAMOMI RAMULUS 74 | SCUTELLARIAE RADIX 149 |
| FORSYTHIAE FRUCTUS 105 | HOUTTUYNIAE HERBA 276 | and GLYCYRRHIZAE RADIX ET RHIZOMA 45. | |

Among the medicinal materials used in the pharmaceutical composition of the present invention, PINELLIAE RHIZOMA is preferably PINELLIAE RHIZOMA PRAEPARATUM CUM ALUMINE, ATRACTYLODIS MACROCEPHALAE RHIZOMA is preferably RHIZOMA ATRACTYLODIS MACROCEPHALAE TOSTUM CUM MELLE ET FURFURE (the Latin name is according to Standard of Chinese Herbal Pieces of Shanxi Province, Feb., 2008), ARMENIACAE SEMEN AMARUM is preferably ARMENIACAE SEMEN AMARUM TOSTUM (the Latin name is according to Standard of Chinese Herbal Pieces of Shanxi Province, Feb., 2008), and SCUTELLARIAE RADIX is preferably in form of slices.

PINELLIAE RHIZOMA and CITRI RETICULATAE PERICARPIUM in the pharmaceutical composition of the present invention are principal drugs. The PINELLIAE RHIZOMA is pungent in taste, warm in nature, and enters the Spleen, Stomach and Lung Channels. Being warm and dry in nature and capable of eliminating dampness by dryness and resolving phlegm, and exerting its efficacy downward, PINELLIAE RHIZOMA is the essential herb for resolving phlegm and descending upward adverse flow of qi and PINELLIAE RHIZOMAalso has cough-checking effect. PINELLIAE RHIZOMA is described as "being able to direct the damp phlegm in the lung and stomach moving downward, absorb qi and relieve asthma" (Yi Xue Zhong Zhong Can Xi Lu). It is also described in Yao Xing Lun that it "disperses phlegm, descends lung qi, increasing appetite and invigorate spleen, and dissipate excessive phlegm and fullness sensation in chest". CITRI RETICULATAE PERICARPIUM is pungent and bitter in taste, warm in nature, acts as the essential herb for qi system of Lung and Spleen Channels, and serves for regulating qi, activating spleen, eliminating dampness and resolving phlegm. Since CITRI RETICULATAE PERICARPIUM is fragrant in flavor and able to move and direct qi downward, it is good at both of regulating qi and eliminating dampness by dryness, thus resulting in invigoration of spleen qi, and thereby extirpates the origin of phlegm . Due to capability of regulating *qi*, drying dampness, resolving phlegm and descending upward adverse flow, the PINELLIAE RHIZOMA and the CITRI RETICULATAE PERICARPIUM are used together as the principal drugs to act predominantly in the whole prescription.

ASTERIS RADIX ET RHIZOMA, FARFARAE FLOS, ATRACTYLODIS MACROCEPHALAE RHIZOMA, PORIA, and ARMENIACAE SEMEN AMARUM in the pharmaceutical composition of the present invention are minister drugs. ASTERIS RADIX ET RHIZOMA is warm and damp in nature, bitter in taste and has a purging action. It exerts good effcts of resolving phlegm and relieving cough. It is described in ShenNong's Herbal that ASTERIS RADIX ET RHIZOMA "is mainly for cough with dyspnea and stagnant qi in chest due to cold and heat". FARFARAE FLOS can moisturize the lung and direct qi downward, relieve cough and resolve phlegm. It is summarized in Ben Jing Feng Yuan that it has functions of "moisturizing the lung and resolving phlegm, checking cough and relieving asthma". ASTERIS RADIX ET RHIZOMA and FARFARAE FLOS are utilized by their mutual reinforcement and are good drugs for treating chronic cough and protracted dyspnea. Both of them have the properties of "being warm but not dry, damp but not greasy", have functions special in warming and moisturizing lung qi, checking cough and resolving phlegm, therefore lung qi is moisturized and pulmonary function of purification and descending is recovered, thereby phlegm can not be accumulated within the lung. Meanwhile, the warm and damp properties of the two drugs can remove the disadvantage of injuring *yin* due to excessive drugs having properties of warm and dry in the prescription. ATRACTYLODIS MACROCEPHALAE RHIZOMA is sweet and bitter in taste, warm in nature. It can be used to replenish *qi* and invigorating spleen, as well as eliminating dampness by dryness while inducing diuresis. In Zhen Zhu Nang, ATRACTYLODIS MACROCEPHALAE RHIZOMAit is described to be good at "removing dampness, benifiting qi, dispersing phlegm and promoting diuresis". PORIA has effects of invigorating spleen, inducing diuresis and removing dampness, and thus specializes in the damp-phlegm cough, as recorded in Shi Bu Zhai Yi Shu: "PORIA as a single drug is primary for treating phlegm. PORIA can alleviate water retention, as the nature of phlegm is water; and can also alleviating dampness retention, as the movement of the phlegm is dampness". The combination of ATRACTYLODIS MACROCEPHALAE RHIZOMA and PORIA results in invigorating spleen, eliminating dampness by dryness, and dispelling phlegm, thus the incidental and fundamental of the disease are treated simultaneously, such that dampness is eliminated and spleen is invigorated, therby phlegm can not generate. ARMENIACAE SEMEN AMARUM is bitter in taste, slightly warm in nature, and has actions of purging with bitter, descending qi, checking cough and relieving dyspnea. After stir-heating (i.e. ARMENIACAE SEMEN AMARUM TOSTUM), ARMENIACAE SEMEN AMARUM not only its toxicity ARMENIACAE SEMEN AMARUM is reduced, but also its function of moisturizing bowels to relieve constipation is reinforced. It is described in Ben Cao Bian Du that: "All of nutlets have the descending action, while apricot seed specializes in descending *qi* which results in dispersing phlegm and checking cough, and further moisturizing large intestine .......". It is also described in Yao Xing Lun that ARMENIACAE SEMEN AMARUM "is mainly for cough with dyspnea". The drugs mentioned above acts together as minister to facilitate the principal drugs to invigorate spleen, eliminate dampness and descend adverse flow of *qi,* so as to resolve phlegm, relieve cough and asthma

PERILLAE FRUCTUS, RAPHANI SEMEN, SINAPIS SEMEN, CINNAMOMI RAMULUS, SCUTELLARIAE RADIX, FORSYTHIAE FRUCTUS, and HOUTTUYNIAE HERBA in the pharmaceutical composition of the present invention are assistant drugs. PERILLAE FRUCTUS is pungent in taste, warm in nature, and has functions of descending qi, dispersing phlegm, relieving cough and asthma. It is described in Ri Hua Zi Ben Cao that it can "relieve cough, moisturize the heart and lung and eliminate phlegme-*qi*." RAPHANI SEMEN is pungent and sweet in taste, netural in nature, enters the Spleen, Stomach, and Lung channels, and has functions of descending qi, dispersing phlegm, treating accumulation and obstruction of phlegm, and panting with cough. It is described in Compendium of Materia Medica that it can "direct qi downward to relieve dyspnea, and treat the phlegm." SINAPIS SEMEN is pungent in taste, warm in nature, and enters Lung channel. SINAPIS SEMENIt has functions of regulating qi with the pungent taste, warming the lung and dispelling phlegm, and can also eliminate the phlegm within the channels and collaterals. SINAPIS SEMEN is used together with PERILLAE FRUCTUS and RAPHANI SEMEN to against accumulation of phlegm and stagnation of *qi* so as to dissolve phlegm by dispersing *qi* and smoothing collaterals, and assist PINELLIAE RHIZOMA to descend upward adverse flow of *qi* and eliminate phlegm. CINNAMOMI RAMULUS is pungent and sweet in taste, and warm in nature. On one hand, by utilizing its pungent taste for dispersing and warm property for getting through channels, CINNAMOMI RAMULUS is able to act on body superficially to relieve exterior syndrome, and thereby to drive pathogenic factors away and smooth collaterals. On the other hand, it is able to make the *yang-qi* in chest getting through channels by its warm property, thus warming and resolving the stasis water and dampness. CINNAMOMI RAMULUS together with PORIA and ATRACTYLODIS MACROCEPHALAE RHIZOMA can warm and promote the spleen *yang,* thus removing dampness and inducing diuresis. SCUTELLARIAE RADIX is bitter in taste and cold in nature, enters the Lung channel, and has functions of clearing heat, eliminating dampness by dryness, and purging fire for detoxication. SCUTELLARIAE RADIX specializes in clearing pulmonary heat, for which Dan Xi had said that "since SCUTELLARIAE RADIX serves for descending the phlegm, it can be used to dissipate fire". FORSYTHIAE FRUCTUS is bitter in taste and cold in nature. It can externally relieve the muscle and the superfacail, and internally clear the stagnant heat, and "has the capacity of lifting and floating as well as dispelling, ...... can relieve exterior syndromes through the muscle, clear heat and expel the wind, so tha t it is the primary drug for treating the disease due to wind-heat pathogen" (Yi Xue Zhong Zhong Can Xi Lu). The heat-clearing and detoxication actions of FORSYTHIAE FRUCTUS is used in the prescription to clear the heat toxin stagnated in collaterals. HOUTTUYNIAE HERBA is pungent in taste and slightly chill in nature, enters lung channel, has functions of clearing heat, detoxication, and exelling pus. It specializes in clearing the pathogenic heat in Lung channel and is the primary drug for treating the pulmonary heat. SCUTELLARIAE RADIX, FORSYTHIAE FRUCTUS and HOUTTUYNIAE HERBA used together in the prescription can clear pulmonary heat and the heat toxin stagnated in collaterals, and prevent phlegm-turbidity from becoming heat.

GLYCYRRHIZAE RADIX ET RHIZOMA in the pharmaceutical composition of the present invention is guide drug. GLYCYRRHIZAE RADIX ET RHIZOMA is used for two purposes: in its crude form for two reasons. The first purpose is that GLYCYRRHIZAE RADIX ET RHIZOMA is used as an assistant drug to invigorate spleen and replenish *qi*, as well as moisturize the lung and relieve cough. The second purpose is that GLYCYRRHIZAE RADIX ET RHIZOMA is used as a guide drug to attemper the drugs of the prescription, thus moderating natrures of the drugs.

In view of the above, the whole prescription of the pharmaceutical composition of the present invention has the characterizations of simultaneous treatment of superficiality and origin of disease, simultaneous application of purgation and reinforcement, and warm without dryness in nature, and combines the actions of regulating *qi*, invigorating spleen, dispelling dampness, resolving phlegm, descending qi, relieving cough and asthma detoxication and the like. Since invigoration of the spleen results in disabling generation of phlegm, and dispelling the phlegm results in spontaneously ventilating the pulmonary qi, cough and dyspnea are relieved. All drugs of the present prescription together attain effects of regulating qi, eliminating dampness, resolving phlegm and relieving cough.

In the light of the present invention the and in combination with the common sense in the field, those skilled in the art may replace the individual components in the pharmaceutical composition of the present invention with other Chinese medicinal materials having same or similar efficacies as the replaced component in the pharmaceutical composition.

The pharmaceutical composition of the present invention can be prepared with conventional preparation methods in the art. For example, the various medicinal substances in the pharmaceutical composition of the present invention can be processed in accordance with the Standard for Processing Traditional Chinese Medicinal Substances or Traditional Chinese Medicine Dictionary.

The pharmaceutical composition of the present invention can be prepared preferably with the following method:
(1) weighing selected clean SCUTELLARIAE RADIX according to the part set forth in the prescription and pulverizing the same into fine powder for later use;
(2) weighing selected clean CITRI RETICULATAE PERICARPIUM and CINNAMOMI RAMULUS according to the parts set forth in the prescription, adding 8-12 folds by v/w (ml/g) of water based on the weight of the medicinal materials, and subjecting the mixture to distillation for 3-7 h to extract essential oils, and collecting the essential oils for later use;
(3) collecting the aqueous solution obtained after the distillation in step (2) in a container, extracting the herbal residue from step (2) together with ATRACTYLODIS MACROCEPHALAE RHIZOMA, PORIA, GLYCYRRHIZAE RADIX ET RHIZOMA, FARFARAE FLOS and FORSYTHIAE FRUCTUS twice with water, each time for 1-3 hours, in which the amount of water is 7-11 times by v/w (ml/g) of the weight of the medicinal materials for the first time and 5-9 times by v/w (ml/g) for the second time; then, pooling the aqueous extracts, and filtrating the same followed by combining the filtrate with the above said aqueous solution obtained after the distillation, and concentrating the mixed solution to a clear paste with the relative density of 1.15-1.20 thermally measured at 60 °C for later ues;
(4) weighing selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the parts set forth in the prescription, extracting the same two times in 5-7 folds by v/w (ml/g) of 40-70% ethanol solution based on the medicinal materials, the first time for 1-3 hours and the second time for 1-2 hours; pooling the extracts and filtrating the same; recovering ethanol in the filtrate, and concentrating the residue to a clear paste with the relative density of 1.15-1.20 thermally measured at 60 °C; and combining the clear paste with the clear paste obtained in step (3) for later use.

The fine powder obtained in step (1), the essential oils obtained in step (2) and the combined clear pastes obtained in step (4) constitutes the active ingredients of the pharmaceutical composition of the present invention.

Dosage forms of the pharmaceutical composition according to the invention include, but are not limited to hard capsules, tablets, powders, oral liquids, soft capsules, pills, tinctures, syrups, suppositorys, gels, sprays, or injections.

The dosage forms of the present medication can be prepared into pharmaceutically acceptable conventional dosage forms with the conventional preparation methods in the art, for example, such as the preparation processes recorded in Pharmaceutics of Chinese Medicine, Fan Biting (Editor), December, 1997(the first edition), Shanghai Scientific & Technical Publishers.

In formulating the various pharmaceuticals of the present invention, a person skilled in the art may add a variety of pharmaceutically acceptable auxiliary materials, for example, including, but not limited to fillers, disintegrants, lubricants, suspending agents, binders, sweeteners, flavoring agents, preservatives, matrixes, or any of mixtures thereof, depending on diferent dosage forms of the pharmaceuticals of the present invention and in combination of the common sense in the art. The fillers include, but are not limited to starch, pregelatinized starch, lactose, mannitol, chitin, microcrystalline cellulose, sucrose, or any of mixtures thereof. The disintegrants include, but are not limited to starch, pregelatinized starch, microcrystalline cellulose, sodium carboxymethyl starch, cross-linked polyvinylpyrrolidone, low substituted hydroxypropyl cellulose, crossl-inked sodium carboxymethyl cellulose, or any of mixtures thereof The lubricants include, but are not limited to magnesium stearate, sodium lauryl sulfate, talc, silica, or any of mixtures thereof The suspending agents include, but are not limited to polyvinylpyrrolidone, microcrystalline cellulose, sucrose, agar, hydroxypropyl methyl cellulose, or any of mixtures thereof. The binders include, but are not limited to starch slurry, polyvinylpyrrolidone, hydroxypropyl methyl cellulose, or any of mixtures thereof The sweeteners include, but are not limited to sodium saccharin, aspartame, sucrose, sodium cyclamate, glycyrrhetinic acid, or any of mixtures thereof The flavoring agents include, but are not limited to sweeteners and various essences, or any of mixtures thereof The preservatives include, but are not limited to: parabens, benzoic acid, sodium benzoate, sorbic acid and salts thereof, benzalkonium bromide, chlorhexidine acetate, eucalyptus oil, or any of mixtures thereof The matrixes include, but are not limited to PEG6000, PEG4000, insect wax, or any of mixtures thereof. In order to prepare the above-described dosage forms according to pharmaceutics of traditional Chinese medicine, other auxiliary materials which are pharmaceutically acceptable (e.g. auxiliary materials for various dosage forms redorded in Pharmaceutics of Chinese Medicine, Fan Biting (Editor), December, 1997(the first edition), Shanghai Scientific & Technical Publishers) my be further added when preparing those dosage forms.

As a preferred embodiment, the pharmaceutical composition of the present invention is prepared with the following method:
(1) weighing selected clean SCUTELLARIAE RADIX according to the part set forth in the prescription and pulverizing the same into fine powder for later use;
(2) weighing selected clean CITRI RETICULATAE PERICARPIUM and CINNAMOMI RAMULUS according to the parts set forth in the prescription, adding 8-12 folds by v/w (ml/g) of water based on the weight of the medicinal materials, subjecting the mixture to distillation for 4-6 h to extract essential oils, and collecting the essential oils for later use;
(3) collecting the aqueous solution obtained after distillation in step (2) by a container; extracting the herbal residue from step (2) together with ATRACTYLODIS MACROCEPHALAE RHIZOMA, PORIA, GLYCYRRHIZAE RADIX ET RHIZOMA, FARFARAE FLOS, and FORSYTHIAE FRUCTUS two times with water, each time for 1-3 hours, in which an amount of water is 7-11 times by v/w (ml/g) of the weight of the medicinal materials for the first time and 5-9 times by v/w (ml/g) for the second time; then, pooling the aqueous extracts and filtrating the mixture, combining the filtrate and the above said aqueous solution obtained after distillation, and concentrating the mixed solution to a clear paste with the relative density of 1.15-1.20 thermally measured at 60 °C for later ues;
(4) weighing selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the parts set forth in the prescription, extracting the same twice in 5-7 folds by v/w (ml/g) of 40-70% ethanol solution based on the weight of the medicinal materials, the first time for 1-3 hours and the second time for 1- hours; pooling the extracts and filtrating the same; recovering ethanol of the filtrate, and concentrating the residue to a clear paste with the relative density of 1.15-1.20 thermally measured at 60 °C, and combining the clear paste with the clear paste obtained in step (3) for later use;
(5) granulating the combined clear pastes obtained in step (4) with the fine powder obtained in step (1) as a fundamental material, and finishing the obtained granules for later use;
(6) providing the materials (parts by weight) for tebleting as follows:
   granules obtained in step (5) 340-600 colloidal silicon dioxide 1.8-3.5
   magnesium stearate 1.8-3.5
(7) adding the essential oils obtained in step (2) to the colloidal silicon dioxide and tableting by a convetional formulation process to obtain the pharmaceutical composition of the present invention.

As a further preferred embodiment, the pharmaceutical composition of the present invention is prepared with the following method:
(1) weighing selected clean SCUTELLARIAE RADIX according to the part set forth in the prescription and pulverizing into fine powder for later use;
(2) weighing selected clean CITRI RETICULATAE PERICARPIUM and CINNAMOMI RAMULUS according to the parts set forth in the prescription, adding 9 folds by v/w (ml/g) of water based on the weight of the medicinal materials, and subjecting the mixture to distillation for 5 h to extract essential oils, and collecting the essential oils for later use;
(3) collecting the aqueous solution obtained after distillation in step (2)in a container, extracting the herbal residue from step (2) together with ATRACTYLODIS MACROCEPHALAE RHIZOMA, PORIA, GLYCYRRHIZAE RADIX ET RHIZOMA, FARFARAE FLOS, and FORSYTHIAE FRUCTUS twice with water, each time for 4 hours, in which an amount of water is 10 times by v/w (ml/g) of the weight of the medicinal materials for the first time and 7 times by v/w (ml/g) for the second time; then, pooling the aqueous extracts and filtrating, combining the filtrate with the above said aqueous solution obtained after distillation, and concentrating the mixed solution to a clear paste with the relative density of 1.17 thermally measured at 60 °C for later ues;
(4) weighing selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the parts set forth in the prescription, extracting twice in 6 folds by v/w (ml/g) of 60% ethanol solution, the first time for 2 hours and the second time for 1 hour; pooling the extracts and filtrating the mixture; recovering ethanol of the filtrate, concentrating the residue to a clear paste with the relative density of 1.17 thermally measured at 60 °C, and combined the clear paste with the clear paste obtained in step (3) for later use;
(5) granulating the combined clear pastes obtained in step (4) with the fine powder obtained in step (1) as a fundamental material, and finishing the obtained granules for later use;
(6) providing the materials (parts by weight) for tableting as follows:
   granules obtained in step (5) 470 colloidal silicon dioxide 2.4
   magnesium stearate 2.4
(7) adding the essential oil obtained in step (2) to the colloidal silicon dioxide and tableting by a convetional formulation process to obtain the pharmaceutical composition of the present invention.

The pharmaceutical composition of the present invention was achieved on the basis of the collateral disease theory from traditional Chinese medicine in combination with the modern medical investigations on the etiology and pathogenesis of the bronchitis, as well as years of the clinical practices. The pharmaceutical composition has effects of regulating *qi*, eliminating dampness by dryness, resolving phlegm and relieving cough, and exerts good efficacies in treating bronchitis, particularly excellent efficacy in treating chronic bronchitis.

It has been shown experimentally that the present pharmaceutical composition has effects of checking cough, dispelling phlegm, relieving asthma and resisting inflammation, and shows particularly excellent efficacy in treating chronic bronchitis. Moreover, the present pharmaceutical composition has been shown experimentally to be free of side effects and safe.

### Detailed Description of the Invention

The present invention will be further illustrated by the following Examples and Test Examples which are not intended to limit the scope of the present invention in any way.

### Example 1 Preparation of tablet of the present medication (tentatively named as: Lian Hua Man Zhi Tablet)

### Prescription:

| | | | |
|---|---|---|---|
| CITRI RETICULATAE PERICARPIUM 94 g | PINELLIAE RHIZOMA 156 g | ASTERIS RADIX ET RHIZOMA 94 g | FARFARAE FLOS 156 g |
| PORIA 94 g | ATRACTYLODIS MACROCEPHALAE RHIZOMA 156 g | ARMENIACAE SEMEN AMARUM 78 g | PERILLAE FRUCTUS 130 g |
| SINAPIS SEMEN 78 g | RAPHANI SEMEN 130 g | CINNAMOMI RAMULUS 46g | SCUTELLARIAE RADIX 94g |
| FORSYTHIAE FRUCTUS 156 g | HOUTTUYNIAE HERBA 391 g | and GLYCYRRHIZAE RADIX ET RHIZOMA 31 g. | |

### Preparation method:

(1)The selected clean SCUTELLARIAE RADIX was weighed according to the weight set forth in the prescription and pulverized into fine powder for later use;
(2) the selected clean CITRI RETICULATAE PERICARPIUM and CINNAMOMI RAMULUS were weighed according to the weights set forth in the prescription, followed by adding water 10 times by v/w (ml/g) of the weight of the medicinal materials, and subjecting to distillation to extract essential oils for 6 h, the essential oils were collected for later use;
(3) the distilled aqueous solution in step (2) was collected by a container, the herbal residue thereof together with ATRACTYLODIS MACROCEPHALAE RHIZOMA, PORIA, GLYCYRRH1ZAE RADIX ET RHIZOMA, FARFARAE FLOS, and FORSYTHIAE FRUCTUS were extracted twice with water, each time for 1.5 hours, in which the amount of water was 10 times by v/w (ml/g) of the weight of the medicinal materials for the first time and 8 times by v/w (ml/g) for the second time; then, the aqueous extracts were pooled, and filtrated, the distilled aqueous solution described above was added into the filtrate, the filtrate was concentrated to obtain a clear paste with the relative density of 1.17 thermally measured at 60°C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 60% ethanol solution in an amount of 7 times in volume (ml) of the weight (g) of the medicinal materials, the first time for 3 hours and the second time for 2 hours; the extracts were pooled and filtrated; ethanol in the filtrate was recovered and the filtrate was concentrated into a clear paste with the relative density of 1.17 thermally measured at 60°C, which was combined with the clear paste obtained in step (3) for later use;
(5) with the fine powder obtained in step (1) as a fundamental material, the combined clear pastes obtained in step (4) were granulated, and finished for later use;
(6) the raw materials used for tablets were weighed according to the following parts by weight:
   granules obtained in step (5) 455 colloidal silicon dioxide 2.2
   magnesium stearate 2.2
(7) the essential oils obtained in step (2) we added to the colloidal silicon dioxide and the subject tablets were prepared by conventional formulation process.

### Example 2 Preparation of capsule of the present medication

### Prescription:

| | | | |
|---|---|---|---|
| CITRI RETICULATE PERICARPIUM 156 g | PINELLIAE RHIZOMA 94 g | ASTERIS RADIX ET RHIZOMA 156 g | FARFARAE FLOS 94 g |
| PORIA 156 g | ATRACTYLODIS MACROCEPHALAE RHIZOMA 94 g | ARMENIACAE SEMEN AMARUM 130 g | PERILLAE FRUCTUS 78 g |
| SINAPIS SEMEN 130 g | RAPHANI SEMEN 78 g | CINNAMOMI RAMULUS 78 g | SCUTELLARIAE RADIX 156 g |
| FORSYTHIAE FRUCTUS 94 g | HOUTTUYNIAE HERBA 225 g | and GLYCYRRHIZAE RADIX ET RHIZOMA 53 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 9 times by v/w (ml/g) of the weight of the medicinal materials, and subjecting to distillation to extract essential oils for 5 h, the essential oils were collected for later use;
(3) the distilled aqueous solution in step (2) was collected by another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 1.5 hours, in which the amount of water was 8 times by v/w (ml/g) of the weight of the medicinal materials for the first time and 6 times by v/w (ml/g) for the second time; then, the aqueous extracts were pooled, filtrated, the distilled aqueous solution described above was added in the filtrate, and the mixture was concentrated into a clear paste with the relative density of 1.16 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 60% ethanol solution in an amount of 5 times in volume (ml) of the weight (g) of the medicinal materials, the first time for 2 hours and the second time for 1.5 hours; the extracts were pooled and filtrated; ethanol in the filtrate was recovered, and the filtrate was concentrated into a clear paste with the relative density of 1.16 thermally measured at 60 °C, and combined with the clear paste obtained in step (3) for later use;
(5) the capsules were prepared with a conventional formulation process.

### Example 3 Preparation of powder of the present medication

### Prescription:

| | | | |
|---|---|---|---|
| CITRI RETICULATAE PERICARPIUM 125 g | PINELLIAE RHIZOMA 125 g | ASTERIS RADIX ET RHIZOMA 125 g | FARFARAE FLOS 125 g |
| PORIA 125 g | ATRACTYLODIS MACROCEPHALAE RHIZOMA 125 g | ARMENIACAE SEMEN AMARUM 104 g | PERILLAE FRUCTUS 104g |
| SINAPIS SEMEN 104 g | RAPHANI SEMEN 104 g | CINNAMOMI RAMULUS | SCUTELLARIAE RADIX |
| | | 62 g | 125 g |
| FORSYTHIAE FRUCTUS 125 g | HOUTTUYNIAE HERBA 313 g | and GLYCYRRHIZAE RADIX ET RHIZOMA 42 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 9 times by v/w (ml/g) of the weight of the medicinal materials, and subjecting to distillation to extract essential oils for 5 h, the essential oils were collected for later use;
(3) the distilled aqueous solution in step (2) was collected into another container, the herbal residue thereof together with the ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 1.5 hours, in which the amount of water was 8 times by v/w (ml/g) of the weight of the medicinal materials for the first time and 6 times by v/w (ml/g) for the second time; then, the aqueous extracts were pooled, filtrated, then the distilled aqueous solution described above was added in the filtrate, and the mixture was concentrated into a clear paste with the relative density of 1.16 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 60% ethanol solution in an amount of 5 times in volume (ml) of the weight (g) of the medicinal materials, the first time for 2 hours and the second time for 1.5 hours; the extracts were pooled and filtrated; ethanol in the filtrate was recovered, and the filtrate was concentrated into a clear paste with the relative density of 1.16 thermally measured at 60 °C, which was combined with the clear paste obtained in step (3) for later use;
(5) the powder was prepared with a conventional formulation process.

### Example 4 Preparation of oral liquor of the present medication

### Prescription:

| | | | |
|---|---|---|---|
| CITRI RETICULATAE PERICARPIUM 98 g | PINELLIAE RHIZOMA 98 g | ASTERIS RADIX ET RHIZOMA 104 g | FARFARAE FLOS 104 g |
| PORIA 144 g | ATRACTYLODIS MACROCEPHALAE RHIZOMA 148 g | ARMENIACAE SEMEN AMARUM 85 g | PERILLAE FRUCTUS 85g |
| SINAPIS SEMEN 125 g | RAPHANI SEMEN 128 g | CINNAMOMI RAMULUS 75 g | SCUTELLARIAE RADIX 151 g |
| FORSYTHIAE FRUCTUS 149 g | HOUTTUYNIAE HERBA 377 g | and GLYCYRRHIZAE RADIX ET RHIZOMA 49 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 8 times by v/w (ml/g) of the weight of the medicinal materials, and subjecting to distillation to extract essential oils for 3 h, the essential oils were collected for later use;
(3) the aqueous solution obtained after distillation in step (2) was collected into another container, the herbal residue thereof together with the ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 1 hour, in which the amount of water was 7 times by v/w (ml/g) of the weight of the medicinal materials for the first time and 5 times by v/w (ml/g) for the second time; then, the aqueous extracts were pooled, filtrated, then the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 40-70% ethanol solution in an amount of 5 times in volume (ml) of the weight (g) of the medicinal materials, the first time for 1 hour and the second time for 1 hour; the extracts were pooled and filtrated; ethanol in the filtrate was recovered, and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the oral liquor by a conventional formulation process.

### Example 5 Preparation of soft capsule of the present medication

### Prescription:

| | | | |
|---|---|---|---|
| CITRI RETICULATAE PERICARPIUM 147 g | PINELLIAE RHIZOMA 152 g | ASTERIS RADIX ET RHIZOMA 149 g | FARFARAE FLOS 140 g |
| PORIA 105 g | ATRACTYLODIS MACROCEPHALAE RHIZOMA 105 g | ARMENIACAE SEMEN AMARUM 81 g | PERILLAE FRUCTUS 81 g |
| SINAPIS SEMEN 125 g | RAPHANI SEMEN 125 g | CINNAMOMI RAMULUS 74 g | SCUTELLARIAE RADIX 149 g |
| FORSYTHIAE FRUCTUS 105 g | HOUTTUYNIAE HERBA 276 g | and GLYCYRRHIZAE RADIX ET RHIZOMA 45 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 12 times by v/w (ml/g) of the weight of the medicinal materials, and subjecting to distillation to extract essential oils for 7 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 3 hour, in which the amount of water was 11 times by v/w (ml/g) of the weight of the medicinal materials for the first time and 9 times by v/w (ml/g) for the second time; then, the aqueous extracts were pooled, filtrated, the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 40-70% ethanol solution in an amount of 7 times in volume (ml) of the weight (g) of the medicinal materials, the first time for 3 hour and the second time for 2 hour; the extracts were pooled and filtrated; ethanol in the filtrate was recovered and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the soft capsules by a conventional formulation process.

### Example 6 Preparation of pill of the present medication

### Prescription:

| | | | |
|---|---|---|---|
| CITRI RETICULATAE PERICARPIUM 98 g | PINELLIAE RHIZOMA 148 g | ASTERIS RADIX ET RHIZOMA 148 g | FARFARAE FLOS 105 g |
| PORIA 102 g | ATRACTYLODIS MACROCEPHALAE RHIZOMA 139 g | ARMENIACAE SEMEN AMARUM 139 g | PERILLAE FRUCTUS 87 g |
| SINAPIS SEMEN 87 g | RAPHANI SEMEN 115 g | CINNAMOMI RAMULUS 115 g | SCUTELLARIAE RADIX 120 g |
| FORSYTHIAE FRUCTUS 118 g | HOUTTUYNIAE HERBA 250 g | and GLYCYRRHIZAE RADIX ET RHIZOMA 49 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 10 times by v/w (ml/g) of the weight of the medicinal materials, and subjecting to distillation to extract essential oils for 5 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 2 hours, in which the amount of water was 8 times by v/w (ml/g) of the weight of the medicinal materials for the first time and 7 times by vlw (ml/g) for the second time; then, the aqueous extracts were pooled, filtrated, then the distilled aqueous solution described above was added in the filtrate, and the mixture was concentrated into a clear paste with the relative density of 1.15-1.20 thermally measured at 60 °C for later use;
(4) the selected clean PINELLIAE. RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 60% ethanol solution in an amount of 6 times in volume (ml) of the weight (g) of the medicinal materials, the first time for 2 hours and the second time for 1.5 hour; the extracts were pooled and filtrated; ethanol in the filtrate was recovered and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the pills by a conventional formulation process.

### Example 7 Preparation of tincture of the present medication

### Prescription:

| | | | |
|---|---|---|---|
| CITRI RETICULATAE PERICARPIUM 148 g | PINELLIAE RHIZOMA 105 g | ASTERIS RADIX ET RHIZOMA 105 g | FARFARAE FLOS 145 g |
| PORIA 145 g | ATRACTYLODIS MACROCEPHALAE RHIZOMA 114 g | ARMENIACAE SEMEN AMARUM 117 g | PERILLAE FRUCTUS 125g |
| SINAPIS SEMEN 125 g | RAPHANI SEMEN 86 g | CINNAMOMI RAMULUS 57 g | SCUTELLARIAE RADIX 144 g |
| FORSYTHIAE FRUCTUS 144 g | HOUTTUYNIAE HERBA 250 g | and GLYCYRRHIZAE RADIX ET RHIZOMA 33 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 11 times by v/w (ml/g) of the weight of the medicinal materials, and subjecting to distillation to extract essential oils for 4 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 1.5 hours, in which the amount of water was 8 times by v/w (ml/g) of the weight of the medicinal materials for the first time and 6 times by v/w (ml/g) for the second time; then, the aqueous extracts were pooled, filtrated, the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 65% ethanol solution in an amount of 5 times in volume (ml) of the weight (g) of the medicinal materials, the first time for 1 hour and the second time for 2 hours; the extracts were pooled and filtrated; ethanol in the filtrate was recovered, and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the tincture by a conventional formulation process.

### Example 8 Preparation of syrup of the present medication

### Prescription:

| | | | |
|---|---|---|---|
| CITRI RETICULATAE PERICARPIUM 99 g | PINELLIAE RHIZOMA 105 g | ASTERIS RADIX ET RHIZOMA 115 g | FARFARAE FLOS 126 g |
| PORIA 137 g | ATRACTYLODIS MACROCEPHALAE RHIZOMA 145 g | ARMENIACAE SEMEN AMARUM 126 g | PERILLAE FRUCTUS 117 g |
| SINAPIS SEMEN 105 g | RAPHANI SEMEN 94 g | CINNAMOMI RAMULUS 60 g | SCUTELLARIAE RADIX 126 g |
| FORSYTHIAE FRUCTUS | HOUTTUYNIAE HERBA | and GLYCYRRHIZAE | |
| 119 g | 300 g | RADIX ET RHIZOMA 39 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 9 times by v/w (ml/g) of the weight of the medicinal materials, and subjecting to distillation to extract essential oils for 6 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 3 hours, in which the amount of water was 10 times by v/w (ml/g) of the weight of the medicinal materials for the first time and 8 times by v/w (ml/g) for the second time; then, the aqueous extracts were pooled, filtrated, the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 55% ethanol solution in an amount of 7 times in volume (ml) of the weight (g) of the medicinal materials, the first time for 2.5 hours and the second time for 2 hours; the extracts were pooled and filtrated; ethanol in the filtrate was recovered, and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the syrup by a conventional formulation process.

### Example 9 Preparation of suppository of the present medication

### Prescription:

| | | | |
|---|---|---|---|
| CITRI RETICULATAE PERICARPIUM 145 g | PINELLIAE RHIZOMA 137 g | ASTERIS RADIX ET RHIZOMA 126 g | FARFARAE FLOS 115 g |
| PORIA 105 g | ATRACTYLODIS MACROCEPHALAE RHIZOMA 99 g | ARMENIACAE SEMEN AMARUM 94 g | PERILLAE FRUCTUS 105g |
| SINAPIS SEMEN 117 g | RAPHANI SEMEN 126g | CINNAMOMI RAMULUS 69 g | SCUTELLARIAE RADIX 119 g |
| FORSYTHIAE FRUCTUS 126 g | HOUTTUYNIAE HERBA 325 g | and GLYCYRRHIZAE RADIX ET RHIZOMA 44 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 10 times by v/w (ml/g) of the weight of the medicinal materials, and subjecting to distillation to extract essential oils for 5 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 1.5 hours, in which the amount of water was 8 times by v/w (ml/g) of the weight of the medicinal materials for the first time and 8 times by v/w (ml/g) for the second time; then, the aqueous extracts were pooled, filtrated, then the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 45% ethanol solution in an amount of 6.5 times in volume (ml) of the weight (g) of the medicinal materials, the first time for 3 hours and the second time for 2 hours; the extracts were pooled and filtrated; ethanol in the filtrate was recovered, and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the suppository by a conventional formulation process.

### Example 10 Preparation of gel of the present medication

### Prescription:

| | | | |
|---|---|---|---|
| CITRI RETICULATAE PERICARPIUM 94 g | PINELLIAE RHIZOMA 125 g | ASTERIS RADIX ET RHIZOMA 119 g | FARFARAE FLOS 123 g |
| PORIA 135 g | ATRACTYLODIS MACROCEPHALAE RHIZOMA 128 g | ARMENIACAE SEMEN AMARUM 115 g | PERILLAE FRUCTUS 108g |
| SINAPIS SEMEN 110 g | RAPHANI SEMEN 130 g | CINNAMOMI RAMULUS 66 g | SCUTELLARIAE RADIX 120 g |
| FORSYTHIAE FRUCTUS 128 g | HOUTTUYNIAE HERBA 290 g | and GLYCYRRHIZAE RADIX ET RHIZOMA 53 g. | |

### Preparation method:

(1)The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 11 times by v/w (ml/g) of the weight of the medicinal materials, and subjecting to distillation to extract essential oils for 5 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 2.5 hours, in which the amount of water was 10 times by v/w (ml/g) of the weight of the medicinal materials for the first time and 8 times by v/w (ml/g) for the second time; then, the aqueous extracts were pooled, filtrated, the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 50% ethanol solution in an amount of 6.5 times in volume (ml) of the weight (g) of the medicinal materials, the first time for 2 hours and the second time for 1.5 hours; the extracts were pooled and filtrated; ethanol in the filtrate was recovered, the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the gel by a conventional formulation process.

### Example 11 Preparation of spray of the present medication

### Prescription:

| | | | |
|---|---|---|---|
| CITRI RETICULATAE PERICARPIUM 138 g | PINELLIAE RHIZOMA 144 g | ASTERIS RADIX ET RHIZOMA 105 g | FARFARAE FLOS 120 g |
| PORIA 131 g | ATRACTYLODIS MACROCEPHALAE RHIZOMA 156 g | ARMENIACAE SEMEN AMARUM 122 g | PERILLAE FRUCTUS 84g |
| SINAPIS SEMEN 119 g | RAPHANI SEMEN 95 g | CINNAMOMI RAMULUS 51 g | SCUTELLARIAE RADIX 95 g |
| FORSYTHIAE FRUCTUS 155 g | HOUTTUYNIAE HERBA 225 g | and GLYCYRRHIZAE RADIX ET RHIZOMA 32 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 10 times by v/w (ml/g) of the weight of the medicinal materials, and subjecting to distillation to extract essential oils for 4 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRHIZAE RADIX ET RHIZOMA, FARFARAE FLOS, and FORSYTHIAE FRUCTUS was extracted twice with water, each time for 2 hours, in which the amount of water was 8 times by v/w (ml/g) of the weight of the medicinal materials for the first time and 8 times by v/w (ml/g) for the second time; then, the aqueous extracts were pooled, filtrated, the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 55% ethanol solution in an amount of 6 times in volume (ml) of the weight (g) of the medicinal materials, the first time for 3 hours and the second time for 1 hour; the extracts were pooled and filtrated; ethanol in the filtrate was recovered, the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the spray by a conventional formulation process.

### Example 12 Preparation of injections of the present medication

### Prescription:

| | | | |
|---|---|---|---|
| CITRI RETICULATAE PERICARPIUM 154 g | PINELLIAE RHIZOMA 95 g | ASTERIS RADIX ET RHIZOMA 149 g | FARFARAE FLOS 106 g |
| PORIA 112 g | ATRACTYLODIS MACROCEPHALAE RHIZOMA 137 g | ARMENIACAE SEMEN AMARUM 121 g | PERILLAE FRUCTUS 109g |
| SINAPIS SEMEN 110 g | RAPHANI SEMEN 115 g | CINNAMOMI RAMULUS 58 g | SCUTELLARIAE RADIX 135 g |
| FORSYTHIAE FRUCTUS 124 g | HOUTTUYNIAE HERBA 294 g | and GLYCYRRHIZAE RADIX ET RHIZOMA 44 g. | |

### Preparation method:

(1) The selected clean SCUTELLARIAE RADIX was weighed according to the weight set forth in the prescription and pulverized into a fine powder for later use;
(2) the selected clean CITRI RETICULATAE PERICARPIUM and CINNAMOMI RAMULUS were weighed according to the weights set forth in the prescription, followed by adding water 10 times by v/w (ml/g) of the weight of the medicinal materials, and subjecting to distillation to extract essential oils for 4 h, the essential oils were collected for later use;
(3) the aqueous solution obtained after distillation in step (2) was collected into another container, the herbal residue thereof together with ATRACTYLODIS MACROCEPHALAE RHIZOMA, PORIA, GLYCYRRH1ZAE RADIX ET RHIZOMA, FARFARAE FLOS, and FORSYTHIAE FRUCTUS was extracted twice with water, each time for 2 hours, in which the amount of water was 8 times by v/w (ml/g) of the weight of the medicinal materials for the first time and 8 times by v/w (ml/g) for the second time; then, the aqueous extracts were pooled, filtrated, the distilled aqueous solution described above was added in the filtrate, and the mixture was concentrated into a clear paste with the relative density of 1.15-1.20 thermally measured at 60 °C for later use;
(4) the selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the weights set forth in the prescription were weighed and extracted twice with 55% ethanol solution in an amount of 6 times in volume (ml) of the weight (g) of the medicinal materials, the first time for 3 hours and the second time for 1 hour; the extracts were pooled and filtrated; ethanol in the filtrate was recovered, and the filtrate was concentrated into a clear paste with the relative density of 1.15-1.20 thermally measured at 60 °C, which was combined with the clear paste obtained in step (3) for later use.

The fine powder obtained in step (1), the essential oils obtained in step (2) and the combined clear pastes obtained in step (4) were formulated into the injections by a conventional formulation process.

All of the pharmaceutical compositions of the present invention administrated in the Test Examples of the following pharmacological and toxicological experiments (hereinafter referred to as "the present medication") were prepared by the procedure set forth in Example 1.

### Test Example 1 Experiment on the cough-checking effect of the present medication

### 1. Cough-checking effect on the aqueous ammonia-induced cough in mice

### 1.1 Experimental materials

### 1.1.1 Medications:

(1) The present medication to be tested, 6.25g crude drugs/g (i.e.,1g of the medication was made from 6.25g crude drugs), provided by Shijiazhuang Yiling Pharmaceutical Co. Ltd., lot number: 060501, and diluted to required concentrations with distilled water in the experiment.
(2) Cloperastine Hydrochloride Tablet (CHT) as the positive control of a western medicine, 10mg/tablet, produced by Beijing Shuguang Pharmaceutical Co., Ltd. lot number: 060619, and valid until June, 2008.
(3) Gui Long Ke Chuan Ning Capsule (GLKCNC) as the positive control of a traditional Chinese medicine, 1g crude drugs/pill (0.3g of powder per pill), produced by Shanxi Guilong pharmaceutical Co., Ltd., Approval number: SFDA Approval No. Z-11; Lot number of the product: 060112; valid until December 2007.

### 1.1.2 Animal:

Sixty Kunming mice of Grade II evenly composed of males and females with the mean body weight of 19.90 ± 0.66 g, provided by Institute of Laboratory Animal Science, Chinese Academy of Medical Sciences, License number: SCXK (Jing) 2000-0006.

### 1.1.3 The Formula of animal feed and nutritional ingredients:

Rat maintenance feed, produced by Beijing Ke Ao Xie Li Feed Co., Ltd., License number: Jing Animal (2000) No.015. Essential components [Beijing Nutrition & Health Inspection File (1997) No.083]: Moisture ≤ 9.5%, ash ≤ 7.8%, protein ≥ 7.8%, fat ≥ 4.3%, crude fiber ≤ 11.0%, lysine ≥ 1.39%, methionine + cysteine: 0.3-0.54%, calcium: 1.0%, phosphorus: 0.54%, folic acid: 21.1mg/kg, vitamin A ≥ 11.4/kg, vitamin D ≥ 98ug/kg, B vitamins: 24.11-164mglkg, calorie: 4.62 thermie/kg.

### 1.1.4 Rear condition for the animals:

Barrier environment rearing facilities of Laboratory Animal Center, Xiyuan Hospital, Chinese Academy of Traditional Chinese Medicine, License number: SCXK (Jing) 2003-0808. Ambient temperature: 22-24°C, Relative humidity: 45-60%.

### 1.1.5 Reagent:

Ammonium hydroxide, produced by Beijing Century Red Star Chemical Co., Ltd. lot number: 20060310.

### 1.2 Experimental process

The sixty healthy mice were randomly assigned into the following six groups: (1) the blank control group administered with distilled water (25 ml/kg); (2) the CHT group (12 mg/kg); (3) the GLKCNC group (2 g crude drug/kg); (4) the low dosage group of the present medication (L-dosage group, 3 g crude drug/kg); (5) the medium dosage group of the present medication (M-dosage group, 6 g crude drug/kg); (6)the high dosage group of the present medication (H-dosage group, 12 g crude drug/kg). The mice were intragastrically administered q.d. at a volume of 25 ml/kg for consecutive three days. Each mouse was placed into a 500 ml beaker containing a tampon in 30 min post the final administration. 0.2 ml of aqueous ammonia was sucked into a 1 ml syringe and injected into the tampon. Then, the beaker was inverted quickly. The mouse was observed to record the cough incubation period and the cough number within 3 minutes. Comparisons among the groups were performed by *t*-test.

### 1.3. Test results

The results are shown in Table 1.

**Table 1: Cough-checking effect on mice**

| Group | Number of animals | Dosage | Incubation period (Sec) | Cough number (times /3 min) |
|---|---|---|---|---|
| | (n) | (/kg) | (*X̅* ±SD) | (*X̅* ±SD) |
| Control group | 10 | 25ml | 60.10±11.70 | 44.80±9.10 |
| CHT group | 10 | 12mg | 118.30±27.23*** | 28.40±9.75** |
| L-dosage group | 10 | 3g | 68.90±21.44 | 40.80±8.27 |
| M-dosage group | 10 | 6g | 79.60±22.24* | 36.00±9.33* |
| H-dosage group | 10 | 12g | 94.80±22.27*** | 31.50±5.99** |
| GLKCNC group | 10 | 2g | 85.40±17.03** | 35.20±7.60* |

| | | | | |
|---|---|---|---|---|
| Note: *P<0.05, **P<0.01, ***P<0.001, as compared to the control group. | | | | |

It can bee seen from Table 1 that both the medium and high dosages of the present medication as well as the positive control of the medication significantly prolong the incubation period of the cough induced by aqueous ammonia in mice and reduce the cough number, and show significant differences as compared to the blank control group (P<0.05, P<0.01, P<0.001).

### 1.4. Summary

The result above shows that the present medication has an effect of significantly prolong the incubation period of the cough induced by aqueous ammonia and reducing the cough number in mice.

### 2. Cough-checking effect on citric acid-induced cough in mice

### 2.1 Experimental materials

### 2.1.1 Medications:

(1) The present medication to be tested, 6.25g crude drugs/g, provided by Shijiazhuang Yiling Pharmaceutical Co. Ltd., lot number: 060501.
(2) GLKCNC as the traditional Chinese medicine control, 1g crude drugs/pill (0.3g of powder per pill), produced by Shanxi Guilong pharmaceutical Co., Ltd., Approval number: SFDA Approval No. Z-11; Lot number of the product: 060112; valid until December 2007.
(3) CHT as the Western medicine control, 10 mg/tablet, produced by Beijing Shuguang Pharmaceutical Co., Ltd., production date: 200606; lot number: 060619; valid until June, 2008.

### 2.1.2 Animal:

Sixty purebred guinea pigs of Grade I evenly composed of males and females with the mean body weight of 206.65 ± 6.92g, provided by Beijing Keyu Animal Breeding Center, License number: SCXK (Jing) 2002-0005.

### 2.1.3 Reagent:

Citric acid, produced by Beijing Chemical Plant, lot number: 960904.

### 2.1.4 Apparatuses:

An air pump, 4L bell-shape glass container, and a glass sparger.

### 2.2 Experimental process

The guinea pigs were individually placed into the sealed 4L bell-shape container with prior to the experiment and a solution of 17.5% citric acid was sprayed for 1 minute at a pressure of 400 mmHg through the glass sparger. The guinea pigs were screened according to the cough number recorded within 5 minutes Guinea pigs with a cough number of less than 10 were rejected and the qualified ones were chosen for the experiment. Sixty screened qualified guinea pigs evenly composed of males and females were randomly assigned into the following six groups: (1) the blank control group administered with distilled water (5 ml/kg); (2) the CHT group (7 mg/kg); (3) the GLKCNC group (1.2g crude drugs/kg); (4) the low dosage group of the present medication (L-dosage group, 1.75 crude drugs/kg); (5) the medium dosage group of the present medication (M-dosage group, 3.5 g crude drugs/kg); (6) the high dosage group of the present medication (H-dosage group, 7 g crude drugs/kg), in which each animals were intragastrically administered q.d. at a volume of 5 ml/kg for consecutive three days, while the animals in the control group were administered with equal amount of physiological saline. Each guinea pig was placed into the sealed 4L bell-shape container in 30 min post the final administration and 17.5% of citric acid was sprayed for 1 minute at a pressure of 600 mmHg through the glass sparger. The guinea pigs were observed to record the cough incubation period and the cough number within 5 minutes. Comparisons among the groups were performed by *t*-test.

### 2.3. Test results

The results are shown in Table 2.

**Table 2: Cough-checking effect on guinea pigs (X̅ ±SD)**

| Group | Number of animals | Dosage | Incubation period | Cough number |
|---|---|---|---|---|
| | (n) | (g/kg) | (sec) | (times/5min) |
| Control group | 10 | 5ml | 86.60±40.07 | 13.80±4.83 |
| CHT group | 10 | 7mg | 196.80±66.22*** | 6.10±3.57*** |
| GLKCNC group | 10 | 1.2 | 152.00±47.56** | 6.90±4.20** |
| L-dosage group | 10 | 1.75 | 109.30t35.14 | 9.30±2.63* |
| M-dosage group | 10 | 3.5 | 138.70±44.87* | 8.20±2.15** |
| H-dosage group | 10 | 7.0 | 181.00±58.73*** | 7.50±3.27** |

| | | | | |
|---|---|---|---|---|
| Note: *P<0.05, **P<0.01, ***P<0.001, as compared to the control group. | | | | |

The experimental results demonstrate that the high and medium dosages of the present medication significantly prolong the cough incubation period and reduce the cough number and show significant differences as compared to the control group (P<0.05, P<0.01, P<0.001). The low dosage of the present medication has a significant effect of reducing the cough number (P<0.05), while hasn't an apparent effect of prolonging the cough incubation period.

### 2.4. Summary

The above-described result indicates that the present medication has an effect of significantly prolonging the cough incubation period and reducing the cough number.

### 3 Conclusions:

The experimental investigations described above show that the present medication has an effect of significantly prolonging the incubation period of the cough induced by aqueous ammonia or citric acid and reducing the cough number in mice, which suggests that the present medication has cough-checking effect.

### Test Example 2 Experiment on phlegm-dispelling effect of the present medication

### 1 Effect on sputum volume in mice measured by phenol red

### 1.1 Experimental materials

### 1.1.1 Medications:

(1) The present medication to be tested, 6.25 g crude drugs/g, provided by Shijiazhuang Yiling Pharmaceutical Co. Ltd., lot number: 060501.
(2) GLKCNC as the traditional Chinese medicine control, 1g crude drugs/pill (0.3g of powder per pill), produced by Shanxi Guilong pharmaceutical Co., Ltd., Approval number: SFDA Approval No. Z-11; Lot number of the product: 060112; valid until December 2007.
(3) Mucosolvan as a western medicine control, 30mg/tablet, the product of Boehringer Ingelheim Int. Ltd. German, lot number: 206362, distribution lot number:030307.

### 1.1.2 Reagent:

(1) Phenol red, 25g/bottle, produced by Beijing Chemical Plant, lot number: 20006017.
(2) NaOH, from Beijing Chemical Reagents Company, lot number: 060901.

### 1.1.3 Animal:

Sixty SPF ICR mice evenly composed of males and females with the mean body weight of 19.02±1.2 g, provided by Vital River Laboratory Animal Technology Co., Ltd., Beijing, and License number: SCXK (Jing) 2002-0003.

### 1.1.4 Instrument:

UV-visible spectrophotometer, model: UV-120-02 , the product from Shimadzu Corporation, Japan.

### 1.2 Experimental process

Sixty healthy mice were randomly assigned into the following six groups: (1) the blank control group administered with distilled water (25 ml/kg); (2) the Mucosolvan group (16 mg/kg); (3) the GLKCNC group (2 g crude drugs/kg); (4) the low dosage group of the present medication (L-dosage group, 3 g crude drugs/kg); (5) the medium dosage group of the present medication (M-dosage group, 6 g crude drugs/kg); (6)the high dosage group of the present medication (H-dosage group, 12 g crude drugs/kg). Each mouse was intragastrically administered q.d. at a volume of 25 ml/kg for consecutive three days. The mice were fasted but had *Ad libitum* access to water prior to the experiment. After the final administration, each mouse was injected 500mg/kg of physiological saline containing 5% phenol red. Thirty minutes later, the mouse was sacrificed and fixed in dorsal position. The skin of neck was incised medianly and the trachea was separated. A tiny opening was cut on the separated trachea and an obtuse syringe needle of 7 Gauge was inserted about 0.3 cm into the trachea through the opening and then ligated firmly with a silk suture. The airway was washed three times with 0.5 ml of physiological saline sucked into a 1 ml syringe and the flush liquid was aspirated into a test tube. This step was repeated 3 times. 0.1ml of 1mol/L NaOH was added to the flush liquid in the tube, allowing the liquid to be basic. The optical density (OD) value of the flush liquid was measured by a UV-visible spectrophotometer UV-120-02 at a wavelength of 546nm, in which the level of phenol red in the flush liquid (µg/ml) was calculated on the basis of phenol red standard curve. Comparisons among the groups were performed by *t*-test.

### 1.3. Test results

The results are shown in Table 3.

**Table 3 Effect on the amount of the phenol red excreted in mice (X̅ ±SD)**

| Group | Dosage | Animal | Amount of excreted phenol red |
|---|---|---|---|
| | (/kg) | (n) | (µg/ml) |
| Control group | 25ml | 10 | 0.507±0.105 |
| GLKCNC group | 2g | 10 | 1.136±0.422*** |
| Mucosolvan group | 16mg | 10 | 1.538±0.486 |
| H-dosage group | 12g | 10 | 1.292±0.683** |
| M-dosage group | 6g | 10 | 1.018±0.605* |
| L-dosage group | 3g | 10 | 0.650±0.384 |

| | | | |
|---|---|---|---|
| Note: *P<0.05; **P<0.01; ***P<0.001, as compared to the control group. | | | |

It can be seen from Table 3 that both the high and medium dosages of the present medication as well as the positive control medication significantly increase the secretion amount, and show significant differences as compared to the control group (P<0.05, P<0.01, P<0.001).

### 1.4. Summary

The above-described results suggest that the present medication has an effect of significantly increasing the excreted sputum volume in mice measured by phenol red.

### 2 Effect on sputum volume in rats measured by capillary

### 2.1 Experimental materials

### 2.1.1 Medications:

(1) The present medication to be tested, 6.25 g of crude drug/g, provided by Shijiazhuang Yiling Pharmaceutical Co. Ltd., lot number: 060501.
(2) GLKCNC as the traditional Chinese medicine control, 1g crude drugs/pill (0.3g of powder per pill), produced by Shanxi Guilong pharmaceutical Co., Ltd., Approval number: SFDA Approval No. Z-11; Lot number of the product: 060112; valid until December 2007.
(3) Mucosolvan as a western medicine control, 30mg/tablet, the product of Boehringer Ingelheim Int. Ltd. German, lot number: 206362, distribution lot number:030307.

### 2.1.2 Animal:

Sixty SPF Wistar mice evenly composed of males and females with the mean body weight of 174.38 ± 7.64 g, provided by Institute of Laboratory Animal Science, Chinese Acadamy of Medical Sciences, and License number: SCXK (Jing) 2000-0006.

### 2.2 Experimental process

The sixty healthy rats were randomly assigned into the following six groups: (1) the blank control group administered with distilled water (10 ml/kg); (2) the Mucosolvan group (11 mg /kg); (3) the GLKCNC group (1.5 g crude drug/kg); (4) the low dosage group of the present medication (L-dosage group, 2 g crude drug/kg); (5) the medium dosage group of the present medication (M-dosage group, 4 g crude drug/kg); (6)the high dosage group of the present medication (H-dosage group, 8 g crude drug/kg). In the experiment, each rat was anesthetized intraperitoneally with 3.5% of chloral hydrate (10ml/kg), laid down on its back horizontally and fixed. The skin of neck was incised and the trachea was separated, on which an orifice was pricked with a syringe needle at the median site of the lower edge of the thyroid cartilage between the two cartilages. A piece of glass capillary with the length of 5 cm and the inner diameter of 0.8 mm was inserted through the orifice in a way such that the glass capillary was just contact the inner wall of the trachea. When the glass capillary was filled up with sputum, it would be replaced immediately with another one. The sputum volume in capillaries was determined in 1 hour prior to the administration. One hour later, the animals in the experimental group were administered duodenally with the medications at a volume of 10 ml/kg, while the animals in the control group were administered with equal amount of physiological saline. All of the animals were observed for the average amount of excreted sputum per hour in 1 or 2 hours post administration. Comparisons among the groups were performed by *t*-test with the average amount of the secretion in 1 hour prior to administration serving as a normal value.

### 2.3. Test results

The results are shown in Table 4.

**Table 4 The effect on excreted sputum volume in rats measured by capillary (X̅ ±SD, n=10)**

| Group | Dosage | | Excretion value (cm) | |
|---|---|---|---|---|
| | (/kg) | 1 h prior to administration | 1 h post administration | 2 h post administration |
| Blank control group | 10ml | 2.54±0.97 | 2.92±1.13 | 2.57±0.98 |
| | | Difference | 0.38±0.67 | 0.03±0.64 |
| | | Change rate(%) | 18.40±30.05 | 3.56±27.60 |
| Mucosolvan group | 11mg | 2.75±1.08 | 8.76±2.51*** | 8.50±1.79*** |
| | | Difference | 6.01±1.56*** | 5.75±1.64*** |
| | | Change rate(%) | 238.05±77.76 | 67.48±11.94 |
| L-dosage group | 2g | 2.89±0.81 | 4.02±0.88* | 3.15±0.89 |
| | | Difference | 1.13±0.77* | 0.26±0.81 |
| | | Change rate(%) | 45.11±35.21 | 12.25±27.55 |
| M-dosage group | 4g | 2.87±0.58 | 4.87±0.61*** | 4.10±1.01** |
| | | Difference | 2.00±0.45*** | 1.23±0.76** |
| | | Change rate(%) | 73.82±25.84 | 27.83±14.75* |
| H-dosage group | 8g | 2.54±0.48 | 5.00±0.65*** | 4.37±0.79*** |
| | | Difference | 2.46±0.81*** | 1.83±0.60*** |
| | | Change rate(%) | 103.98±49.49*** | 74.08±26.71 |
| GLKCNC group | 11g | 2.53±0.70 | 4.36±2.04 | 5.28±1.73*** |
| | | Difference | 2.01±1.64** | 2.93±1.44*** |
| | | Change rate(%) | 87.31±62.42 | 132.80±76.71 |

| | | | | |
|---|---|---|---|---|
| Note: *P<0.05, **P<0.01, ***P<0.001, as compared to the control group. | | | | |

It can be seen from Table 4 that the high, medium, and low dosages of the present medication as well as the positive control medication significantly increase the secretion amount from the respiratory tract of rats, and show significant differences as compared to the control group (P<0.05, P<0.01, P<0.001).

### 2.4. Summary

The above-described results suggest that the present medication has an effect of significantly increasing the secretion amount from the respiratory tract of rats.

### 3 Conclusion:

The two experimental investigations described above confirm that the present medication has an effect of dispelling phlegm.

### Test Example 3 Experiment on dyspnea-relieving effect of the present medication

### 1 Dyspnea-relieving effect of the present medication on the panting in the guinea pig induced by histamine plus acetylcholine

### 1.1 Experimental materials

### 1.1.1. Medications:

(1) The present medication to be tested, 6.25 g crude drugs/g, provided by Shijiazhuang Yiling Pharmaceutical Co. Ltd., lot number: 060501.
(2) GLKCNC as the traditional Chinese medicine control, 1g of crude drug/pill (0.3g of powder), produced by Shanxi Guilong pharmaceutical Co., Ltd., Approval number: SFDA Approval No. Z-11; Lot number of the product: 060112; valid until December 2007.
(3) Aminophylline as a western medicine control, 0.1g/tablet, the product from Beijing Zizhu Pharmaceutical Co., Ltd, lot number: 20061202.

### 1.1.2. Animals:

Sixty white purebred guinea pigs of Grade I evenly composed of males and females with the mean body weight of 198.55 ± 6.30g, provided by Beijing Keyu Animal Breeding Center, License number: SCXK (Jing) 2002-0005.

### 1.1.3 Reagent:

(1) Acetylcholine chloride, 1g/bottle, from Beijing Chemical Reagents Company, lot number: 060211.
(2) Histamine phosphate, 5g/bottle, from Shanghai Institutes of Biological Science, Chinese Academy of Sciences, Production No. 1702.

### 1.1.4 Instrument:

An asthma-inducing apparatus, an air compressor, a glass nebulizer, and a 4L bell-shape glass container.

### 1.2 Experimental process

The healthy guinea pigs evenly composed of males and females were screened preciously. The guinea pigs were individually placed into the 4 L bell-shape glass container and sprayed with a mixed liquid of 2 % acetylcholine chloride and 0.1 % histamine phosphate in equal volumes at a pressure of 400 mmHg for 20s. After the spaying, the guinea pigs were observed for the incubation period for asthmatic convulsion (the time that the asthmatic convulsion takes to occur after ceasing the spaying) within 6 minutes. The guinea pig showing the incubation period for asthmatic convulsion above 120s would not be chosen. Sixty screened guinea pigs were randomly assigned into the following six groups with 10 animals per group: (1) the control group administered with distilled water (5 ml/kg); (2) the Aminophylline group (0.05 g/kg); (3) the GLKCNC group (1.2 g crude drug/kg); (4) the low dosage group of the present medication (L-dosage groups, 1.75g crude drugs/kg); (5) the medium dosage group of the present medication (M-dosage group, 3.5 g crude drugs/kg); (6) the high dosage group of the present medication (H-dosage group, 7 g crude drugs/kg). The animals in the test groups were intragastrically administered q.d. at a volume of 5 ml/kg for consecutive three days, while the animals in the control group were administered with equal amount of physiological saline. An asthma-inducing experiment was performed in 30 min post the final administration. The guinea pigs were individually placed into the 4 L bell-shape glass container and sprayed with a mixed liquid of 2 % acetylcholine chloride plus 0.1 % histamine phosphate in equal volumes at a pressure of 400 mmHg for 20s. Then, the guinea pigs were observed for the incubation period for asthmatic convulsion (also known as the asthma-inducing incubation period) within 6 minutes, that is, the time it takes for asthmatic convulsion to occur and for the guinea pig to fall down after ceasing the spaying. The incubation period lasting for more than 6 min was counted as 360 s. The experimental results were compared among the groups by *t*-test.

### 1.3. Test results

The results are shown in Table 5.

**Table 5 Effect on dyspnea of guinea pigs induced by histamine plus acetylcholine (n=10 , X̅ ±SD)**

| Group | Dosage | Incubation period | Falling-down time |
|---|---|---|---|
| | (g/kg) | (second) | (second) |
| Control group | 5ml | 68.6±42.2 | 129.4±49.5 |
| Aminophylline group | 0.05 | 228.0±43.3*** | 358.5±3.4*** |
| L-dosage group | 1.75 | 94.7±29.6 | 215.4±53.8** |
| M-dosage group | 3.5 | 116.0±30.9* | 242.2±64.2*** |
| H-dosage group | 7 | 145.8±45.5** | 266.5±58.2*** |
| GLKCNC group | 1.2mg | 123.3±37.0** | 215.1±110.2* |

| | | | |
|---|---|---|---|
| Note: *P<0.05; **P<0.01; ***P<0.001, as compared to the control group. | | | |

It can bee seen from Table 5 that both the medium and high-dosage of the present medication can significantly prolong the incubation period and deferring the falling-down of guinea pigs with asthma induced by acetylchline chloride plus histamine phosphate in comparison with the control group (P<0.05, P<0.01, P<0.001). The low dosage of the present medication can significantly defer the falling-down in comparison with the control group (P<0.05).

### 1.4. Summary

The experimental results above show that the present medication has a significant dyspnea-relieving effect on asthma of guinea pigs induced by acetylchline chloride plus histamine phosphate.

### 2. Effect on ovalbumin-induced allergic bronchial spasm in guinea pigs

### 2.1 Experimental materials

### 2.1.1. Medications:

(1) The present medication to be tested, 6.25 g crude drugs/g, provided by Shijiazhuang Yiling Pharmaceutical. Co. Ltd., lot number: 060501, the powdered extract thereof was used and diluted into the required concentrations in the experiment.
(2) GLKCNC as a traditional Chinese medicine control, 1g crude drugs/pill (0.3g of powder per pill), produced by Shanxi Guilong pharmaceutical Co., Ltd., Approval number: SFDAApproval No. Z-11; production date: 2006.01.01; lot number of the product: 060112; valid until December 2007.
(3) Aminophylline as a western medicine control, 0.1g/tablet, the product from Beijing Zizhu Pharmaceutical Co., Ltd, lot number: 20061202.

### 2.1.2. Animals:

Sixty white purebred guinea pigs of Grade I evenly composed of males and females with the mean body weight of 222.33 ± 20.0g, provided by Beijing Keyu Animal Breeding Center, License number: SCXK (Jing) 2002-0005.

### 2.1.3 Reagents:

(1) Antigen: Ovalbumin, 50g /bottle, from Sigma, Cat: A5253, packinged by Beijing Xin Hui Ze Ao Science and Technology Co., Ltd.
(2) Adjuvant: Pertussis vaccine, thirty billion units/ml, 2ml/ampule, from National Institute of Control on Pharmaceutical and Biological Product, lot number: 04-1.

### 2.1.4 Instrument:

An asthma-inducing apparatus, an air compressor, a glass nebulizer, and a 4L bell-shape glass container.

### 2.2 Experimental process

The experiment was performed in two stages. Firstly, the guinea pigs were sensitized. Sixty white, healthy and purebred guinea pigs were selected and each of them was injected intramuscularly at the rear leg with 4 mg of ovalbumin (0.1ml of physiological saline containing 4% ovalbumin) and at same time injected intraperitoneally with 2×10¹⁰ bacteria of the pertussis vaccine, for sensitization. The sensitized animals were used in the experiment in 14 days post the injection. Secondly, the animals at Day 10 of the sensitization were randomly assigned into the following six groups with 10 animals per group: (1) the blank control group administered with distilled water (5ml/kg); (2) the Aminophylline group (0.05g/kg); (3) the GLKCNC group (1.2g crude drugs/kg); (4) the low dosage group of the present medication (L-dosage group, 1.75g crude drugs/kg); (5) the medium dosage group of the present medication (M-dosage group, 3.5g crude drugs/kg); (6) the high dosage group of the present medication (H-dosage group, 7g crude drugs/kg). The animals in the test groups were intragastrically administered at a volume of 5 ml/kg q.d. for consecutive five days, while the animals in the blank group were administered with equal volume of physiological saline. The guinea pigs were individually placed into the sealed 4L bell-shape container in 30 min post the final administration and sprayed with a solution of 5 % ovalbumin at the constant pressure of 400 mmHg for half of a minute. The sprayed guinea pigs were observed to record the incubation period of asthmatic convulsion and the numbers of the animals displaying dyspnea, convulsion and falling-down as well as death within 6 minutes (360 seconds). For the animal without dyspnea and without convulsion and falling-down within 6 minutes, the incubation period thereof was counted as 360 s. The experimental results were compared among the groups by *t*-test. The number of the dead animals was examined by Chi-square-test.

### 2.3. Test results

The results are shown in Table 6.

**Table 6 Effect on ovalbumin-induced allergic bronchial spasm in guinea pigs (n=10, X̅ ±SD)**

| Group | Dosage | Incubation period | Occurrence of dyspnea | Occurrence of convulsion and fall-down | Number of dead animals |
|---|---|---|---|---|---|
| | (g/kg) | (second) | (second) | (second) | |
| Blank control group | 5ml | 51.30±13.51 | 73.70±25.72 | 101.50±40.07 | 9 |
| Aminophylline group | 0.05 | 330.00±51.69*** | 352.50±23.72*** | 360.00±0.00*** | 0*** |
| L-dosage group | 1.75 | 105.20±46.30** | 142.90±72.30* | 196.80±111.64* | 5 |
| M-dosage group | 3.5 | 123.50±43.90*** | 167.60±51.53*** | 249.60±97.03*** | 3* |
| H-dosage group | 7 | 188.50±77.64*** | 257.00±96.84*** | 306.00±85.04*** | 2* |
| GLKCNC group | 1.2mg | 84.60±32.17** | 165.80±78.91** | 255.00±103.57*** | 3* |

| | | | | | |
|---|---|---|---|---|---|
| Note: *P<0.05; **P<0.01; ***P<0.001. as compared to the control group. | | | | | |

It can bee seen from Table 6 that the low, medium and high dosages of the present medication as well as the positive control medication can prolong the asthma incubation period, defer occurrence of dyspnea, convulsion and fall-down, and reduce the number of dead animals, and show significant differences as compared to the control group (P<0.05, P<0.01, P<0.001).

### 2.4. Summary

The experimental results show that the low, medium and high dosages of the present medication can prolong the incubation period of the allergic bronchial spasm and defer the occurrence of dyspnea, convulsion and fall-down, and can also reduce the number of dead animal, thus significantly ameliorating the ovalbumin-induced allergic bronchial spasm.

### 3 Conclusion:

The two experimental investigations described above suggest that the present medication has an effect of relieving dyspnea.

### Test Example 4 Anti-inflammation experiment of the present medication

### 1. Effect on xylene-induced ear swelling in mice

### 1.1. Experimental materials

### 1.1.1. Animals:

Sixty Kunming male mice of Grade II with the mean body weight of 19.77 ± 0.91 g, provided by the breeding farm of the Institute of Laboratory Animal Science, Chinese Academy of Medical Sciences, License number: SCXK (Jing) 2000-0006.

### 1.1.2. Medications:

(1) The present medication to be tested, 6.25 g crude drugs/g, provided by Shijiazhuang Yiling Pharmaceutical Co. Ltd., lot number: 060501.
(2) GLKCNC as a traditional Chinese medicine control, 1g crude drugs/pill (0.3g of powder per pill), produced by Shanxi Guilong pharmaceutical Co., Ltd., Approval number: SFDA Approval No. Z-11; Lot number of the product: 060112; valid until December 2007.
(3) Aspirin enteric-coated tablet as a western medicine control, 0.3 g/tablet, produced by Yantai the Second Pharmaceutical Factory), lot number: 011102. The above-described medications were formulated with distilled water into the required concentrations in the experiment.

### 1.2. Experimental process

Sixty healthy mice were randomly assigned into the following six groups with 10 animals per group: (1) the control group administered with physiological saline (25 ml/kg); (2) the Aspirin group (0.2 g/kg); (3) the GLKCNC group (2 g crude drugs/kg); (4) the low dosage group of the present medication (L-dosage group, 3g crude drugs/kg); (5) the medium dosage group of the present medication (M-dosage group, 6g crude drugs/kg); (6)the high dosage group of the present medication (H-dosage group, 12g crude drugs/kg). The animals in the test groups were intragastrically administered at a volume of 25 ml/kg q.d. for consecutive three days, while the animals in the blank group were administered with equal volume of physiological saline. 0.1 ml of xylene was dropped onto the right ear of each mouse in 30 minutes after the administration at Day 3. Fifteen minutes later, the animal was sacrificed by dislocation. Holes were punched with a hole puncher of 6 mm at the same sites on the right and left auricles. The pieces from the right and left ears were weighted respectively on an electronic balance. The degree of ear swelling for each mouse was calculated by subtraction of the weight of the left piece from that of the right piece. Comparisons of the degree of ear swelling among the groups were performed by *t*-test.

### 1.3. Test results

The results are shown in Table 7.

**Table 7 Effect of the present medication on xylene-induced mouse ear swelling (n=10, X̅ ±SD)**

| Group | Dosage | Number of animals | Swelling rate |
|---|---|---|---|
| | (g/kg) | | ( mg ) |
| Blank control group | 25ml/kg | 10 | 7.50±2.07 |
| Aspirin qroup | 0.2 | 10 | 3.50±0.71*** |
| GLKCNC group | 2 | 10 | 4.80±1.87** |
| L-dosage group | 3 | 10 | 6.20±2.25 |
| M-dosage group | 6 | 10 | 5.30±1.77* |
| H-dosage group | 12 | 10 | 5.00±1.25** |

| | | | |
|---|---|---|---|
| Note: *P<0.05; **P<0.01; ***P<0.001, as compared to the control group. | | | |

As shown in Table 7, the right ears of the mice in the control group swell significantly with increased thickness and differences between the pieces from the right and left ears is obvious. The high and medium dosages of the present medication as well as the positive control medication can significantly inhibit the xylene-induced ear inflammation in mice as the swelling of right ears in mice reduced significantly and show significant differences as compared to the control group (P<0.05, P<0.01), while the low dosage of the present medication doesn't show apparent swelling-inhibitory effect and displays no significant difference as compared to the control group. The experimental results show that the medium and high dosages of the present medication have better anti-inflammation effect on the xylene-induced ear swelling mouse model.

### 1.4. Summary

The experimental results show that the medium and high dosages of the present medication exert significant anti-inflammation effect on the xylene-induced ear swelling mouse model.

### 2. Effect on carrageenan-induced swelling of rat's footpad

### 2.1. Experimental materials

### 2.1.1. Medications:

(1) The present medication to be tested, 6.25g crude drugs/g, provided by Shijiazhuang Yiling Pharmaceutical Co. Ltd., lot number: 060501.
(2) GLKCNC as a traditional Chinese medicine control, 1g crude drugs/pill (0.3g of powder), produced by Shanxi Guilong pharmaceutical Co., Ltd., Approval number: SFDA Approval No. Z-11; Lot number of the product: 060112; valid until December 2007.
(3) Aspirin enteric-coated tablet as a western medicine control, 0.3 g/tablet, produced by Yantai the Second Pharmaceutical Factory), lot number: 011102.
   The above-described medications were prepared into the required concentrations with distilled water in the experiment.
(4) Carrageenan, from SIGMA, lot number: 117H0151, prepared into required concentrations for the experiment.

### 2.1.2. Animals:

Sixty while Wistar male rats of Grade I with the mean body weight of 157.05 ± 8.49 g, provided by Institute of Laboratory Animal Science, Chinese Academy of Medical Sciences, License number: SCXK (Jing) 2000-0006.

### 2.2 Experimental process

The sixty healthy rats were randomly assigned into the following six groups: (1) the blank control group administered with distilled water (10 ml/kg); (2) the Aspirin group (150 mg/kg); (3) the GLKCNC group (1.5 g crude drugs/kg); (4) the low dosage group of the present medication (L-dosage group, 2 g crude drugs/kg); (5) the medium dosage group of the present medication (M-dosage group, 4 g crude drugs/kg); (6)the high dosage group of the present medication (H-dosage group, 8 g crude drugs/kg). The volume of the left hind foot of each animal determined by capillary amplification method was used as the value prior to administration. The animals in the test groups were intragastrically administered at a volume of 10 ml/mg q.d. for consecutive three days, while the animals in the control group were intragastrically administered with the equal volume of physiological saline. Each rat was inflamed by subcutaneous injection 0.05 ml of 1% carrageenan into the rat left footpad in 30 min post the final administration. The volumes (ml) of the rat left hind foot were determined at 0.5, 1, 2, 4, and 6 hours after inflammation. Comparisons among the groups were performed on the difference between the left foot volumes before and after inflammation by *t*-test.

### 2.3. Test results

The results are shown in Table 8.

**Table 8 Effect on carrageenan-induced swelling of rat's footpad (n=10, X̅ ±SD)**

| Group | Dosage | Before inflammation | | | Changes of foot volume after inflammation (ml) | | |
|---|---|---|---|---|---|---|---|
| | (g/gk) | Foot volume (ml) | 0.5h | 1h | 2h | 4h | 6h |
| Control group | | 0.96±0.03 | 1.30±0.06 | 1.37±0.04 | 1.42±0.04 | 1.42±0.05 | 1.42±0.04 |
| | | Difference | 0.35±0.07 | 0.41±0.05 | 0.46±0.05 | 0.46±0.07 | 0.46±0.05 |
| | | Swelling rate (%) | 36.60±8.62 | 43.25±6.48 | 48.47±6.41 | 48.32±8.27 | 48.26±6.22 |
| Aspirin group | 0.15 | 0.98±0.03 | 1.19±0.04 | 1.23±0.04 | 1.27±0.02 | 1.30±0.04 | 1.24±0.03 |
| | | Difference | 0.21±0.05*** | 0.25±0.03*** | 0.29±0.03*** | 0.32±0.04*** | 0.26±0.04*** |
| | | Swelling rate (%) | 21.4±5.49*** | 25.4±3.87*** | 29.7±4.00*** | 32.80±4.78*** | 26.26±3.95*** |
| | | Inhibition rate (%) | 41.53 | 41.19 | 38.63 | 32.11 | 45.59 |
| GLKCNC group | 1.5 | 0.98±0.04 | 1.25±0.06 | 1.31±0.04 | 1.36±0.04 | 1.37±0.06 | 1.31±0.06 |
| | | Differenc e | 0.27±0.06* | 0.33±0.04*** | 0.38±0.04*** | 0.29±0.05*** | 0.33±0.06* |
| | | Swelling rate (%) | 27.29±6.41* | 33.2±4.27*** | 38.61±5.29** | 39.85±6.84* | 33.72±6.88*** |
| | | Inhibition rate (%) | 25.41 | 23.06 | 20.35 | 17.52 | 30.13 |
| L-dosage group | 2 | 0.98±0.03 | 1.23±0.03 | 1.30±0.03 | 1.33±0.03 | 1.40±0.02 | 1.35±0.02 |
| | | Differenc e | 0.25±0.03** | 0.32±0.03*** | 0.35±0.02*** | 0.29±0.07*** | 0.3810.04*** |
| | | Swelling rate(%) | 25.85±3.70** | 33.0±3.70*** | 36.2±3.20*** | 43.48±4.27 | 38.49±5.10** |
| | | Inhbition rate (%) | 29.35 | 23.66 | 25.16 | 10.03 | 20.25 |
| M-dosage group | 4 | 0.98±0.04 | 1.16±0.05 | 1.24±0.04 | 1.29±0.04 | 1.33±0.03 | 1.27±0.03 |
| | | Differenc e | 0.23±0.03*** | 0.27±0.03*** | 0.32±0.02*** | 0.36±0.02*** | 0.28±0.02*** |
| | | Swelling rate (%) | 24.0±2.82*** | 28.4±2.57*** | 32.8±2.08*** | 37.18±2.99*** | 28.86±2.53*** |
| | | Inhibition rate (%) | 34.26 | 34.31 | 32.31 | 23.06 | 40.19 |
| H-dosage group | 8 | 0.97±0.04 | 1.14±0.03 | 1.21±0.02 | 1.25±0.04 | 1.30±0.03 | 1.24±0.03 |
| | | Differenc e | 0.23±0.04*** | 0.30±0.07*** | 0.36±0.06*** | 0.2±0.11*** | 0.35±0.10** |
| | | Swelling rate (%) | 22.9±4.21*** | 31.04±7.81** | 36.53±7.28** | 41.33±10.17 | 35.44±11.02*** |
| | rate (%) | Inhibition rate (%) | 37.32 | 28.24 | 24.63 | 14.46 | 26.57 |

| | | | | | | | |
|---|---|---|---|---|---|---|---|
| Note: *P<0.05; ** P<0.01; ***P<0.001, as compared to the control group. | | | | | | | |

As shown in Table 8, the low, medium and high dosages of the present medication as well as the positive control medication have significant effect on inhibition of swelling of rat's foot at each time points of 0.5, 1, 2, 4, and 6 hours after inflammation and show significant differences as compared to the control group (P<0.05, P<0.01, P<0.001).

### 2.4. Summary

The results suggest that the low, medium and high dosages of the present medication exert an effect of significantly inhibiting the carrageenan-induced swelling inflammation of rat's footpad.

### 3. Effect on samall tampon-induced granuloma in rats

### 3.1. Experimental materials

### 3.1.1. Animals

Sixty Wistar mice of Grade II evenly composed of males and females with the mean body weight of 132.37 ± 6.31 g, provided by the breeding farm of the Institute of Laboratory Animal Science, Chinese Acadamy of Medical Sciences, and License number: SCXK (Jing) 2000-0006.

### 3.1.2 Medications

(1) The present medication to be tested, 6.25 g crude drugs/g, provided by Shijiazhuang Yiling Pharmaceutical Co. Ltd., lot number: 060501.
(2) GLKCNC as a traditional Chinese medicine control, 1g of crude drugs/pill (0.3g of powder), produced by Shanxi Guilong pharmaceutical Co., Ltd., Approval number: SFDAApproval No. Z-11; Lot number: 060112.
(3) Aspirin Enteric-coated Tablet as a western medicine control, 0.3 g/tablet, produced by Yantai the Second Pharmaceutical Factory), lot number: 051102. The medication was prepared to required concentrations with distilled water in the experiment.
(4) Ampicillin Sodium Injection (ASI), 0.5 g/bottle, Produced by North China Pharmaceutical Co., Ltd. lot number: F0111207.

### 3.2 Experimental process

The sixty healthy rats were randomly assigned into the following six groups with 10 animals per group: (1) the blank control group administered with distilled water (10 ml/kg); (2) the Aspirin group (150 mg/kg); (3) the GLKCNC group (1.5 g crude drugs/kg); (4) the low dosage group of the present medication (L-dosage group, 2g crude drugs/kg); (5) the medium dosage group of the present medication (M-dosage group, 4g crude drugs/kg); (6)the high dosage group of the present medication (H-dosage group, 8g crude drugs/kg). In the experiment, the rat was intraperitoneally with 3.5% of chloral hydrate, had its axillary fur been shaved, and fixed in dorsal position. The skin of axilla was disinfected routinely and then incised to form an incision. Through the incision, a weighed small tampon (about 30mg of each cotton ball), which had been autoclaved and added with 1mg/0.1ml ampicillin sodium and then dried in an oven at 50°C, were implanted subcutaneously into the left and right axilla, respectively. Administration was performed 2 hours post the surgery. The animals in the test groups were intragastrically administered q.d. at a volume of 10 ml/kg for consecutive seven days, while the animals in the control group were administered with equal amount of physiological saline. The animals sacrificed by cervical dislocation in 7 days post administration. The tampons were removed to weigh their wet weights on an electronic balance, and then placed in an oven at 60°C for 12 h, followed by weighing the dry weights thereof. The average wet and dry weights of the post-inflammation granuloma for each of the groups were calculated, from which the original weight of the cotton ball had been subtracted, and were compared among the groups by *t*-test.

### 3.3. Test results

The results are shown in Table 9.

**Table 9 Effect on samll tampon-induced granuloma in rats (X̅±SD)**

| Group | Dosage | Number of animals | Wet weight | Dry weight |
|---|---|---|---|---|
| | (g/kg) | (n) | (mg) | (mg) |
| Blank group | 25ml/kg | 10 | 611.95±68.09 | 129.65±11.48 |
| Aspirin group | 0.15 | 10 | 457.85±50.01*** | 99.20±10.16*** |
| GLKCNC group | 1.5 | 10 | 493.65±47.91*** | 111.40±8.02** |
| L-dosage group | 2 | 10 | 511.90±51.34** | 117.45±10.02* |
| M-dosage group | 4 | 10 | 457.75±47.40*** | 106.20±13.78** |
| H-dosage group | 8 | 10 | 430.90±47.90*** | 102.65±12.27*** |

| | | | | |
|---|---|---|---|---|
| Note: *P<0.05; ** P<0.01; ***P<0.001, as compared to the control group. | | | | |

As shown in Table 9, the low, medium, and high dosages of the present medication as well as the positive control medication can apparently inhibite the wet and dry weights of the tampon-induced granuloma in rats and show significant differences as compared to the control group (P<0.05, P<0.01, P<0.001). The results show that the low, medium, and high dosages of the present medication exert an apparent effect on inhibition of the granuloma in the tampon-induced inflammation rat model.

### 3.4. Summary

The experimental results show that the present medication has better anti-inflammatory effect on the rat inflammation model of tampon-induced granuloma.

### 4 Conclusion:

The three experimental investigations described above confirm that the present medication has an anti-inflammatory effect.

### Test Example 5 Effect of the present medication on animal model of chronic bronchitis

### 1 Experimental materials

### 1.1. Medications:

(1) The present medication, 6.25g crude drugs/g, provided by Shijiazhuang Yiling Pharmaceutical Co. Ltd., lot number: 060401, used in the form of powder of extract thereof in the experiment.
(2) GLKCNC, 1g crude drugs/pill (0.3g of powder per pill), produced by Shanxi Guilong pharmaceutical Co., Ltd., Approval number: SFDAApproval No. Z-11; production date: 2006.01.01; lot number of the product: 060112; valid until December 2007.

### 1.2. Animals:

One hundred healthy SPF KM mice, evenly composed of males and females with the body weight of 18-20 g, provided by the breeding farm of the Institute of Laboratory Animal Science, Chinese Academy of Medical Sciences, License number: SCXK (Jing) 2004-0001.

### 1.3. Instrument:

Plastic boxes 40×30×22(cm).
SN-6110 Radioimmunoassay y Counter (having probe), produced by Shanghai Hesuo Rihuan Photoelectric Instrument Co., Ltd.

### 1.4. Reagent:

Interleukin-6 Radioimmunoassay kit (IL-6RIA KIT), Lot number of the product: IF₁ 501, produced by Tianjin Nine Tripods Medical & Bioengineering Co., Ltd.

### 2 Experimental processes

Replication of animal model: Before the experiment, the mice were evenly arranged in the plastic boxes (40×30×22cm). Each box had a ventilating hole and communicated with a three-way pipe which was connected with a 50 ml syringe on one end and with a burning cigarette on the other one (the cigarette is Qian Men non-filter-tip cigarette, produced by Shanghai Cigarette Factory). Through the three-way pipe, cigarette fume was continuously pumped into the box by the syringe. The animals were fumed twice a day in the morning and in the afternoon respectively, and each time for 30 min with two cigarettes. After the experiment was conducted for 6 weeks, several mice were selected randomly and sacrificed. The lungs of the sacrificed mouse were immediately removed, fixed in 10% formalin, and HE-stained. After lesions such as inflammatory infiltration into bronchi, detachment of epithelial cells, etc. were found via pathological observation under a microscope, the fumed mice were randomly assigned into six groups with 15 animals per group at Week 8: (1) the normal control group (distilled water of 25ml/kg); (2) the model group (distilled water 25ml/kg + fumed); (3) GLKCNC (2g crude drugs/kg) as the positive medication + fumed group; (4) the present medication (3 g crude drugs/kg) + fumed group; (5) the present medication (6 g crude drugs/kg) + fumed group; (6) the present medication (12 g crude drugs/kg) + fumed group. The animals in the medications groups were intragastrically administered at a volume of 25 ml/kg q.d. for consecutive four weeks. The animals in the control group and the model group were intragastrically administered with the equal volume of physiological saline. While administered, the animals in the medications groups were fumed (according to the same procedure as the above) over total of 11 weeks. During the course of this period, the animals were observed daily in respect of appearances, diet and death. Eleven weeks later, animals in the groups were subjected to orbital sinus bleeding. Serum of each sample was isolated and centrifugated at 3000 rpm for 5 min. The supernatant was collected to determine interleukin-6. The inflammatory response and function of anti-infection defense within the body of the mice were observed by determining the levels of interleukin-6. Then, the animals were sacrificed. The lungs were immediately removed, fixed in 10% formalin, sliced histologically, HE-stained and observed for pathological changes. (1) Each slice was observed for the degree of inflammatory cell infiltration into the periphery of bronchi and bronchioles of various divisions in the lung and ranked to three grades: +, ++ and +++, according to the portion of the area of inflammatory cell infiltrated bronchi and bronchioles of various divisions over the whole slice and the counts of the inflammatory cells. (2) Each slice was evaluated by observation for the degree of detachment of epithelial cells from all bronchial mucus membrane and ranked to three grades: -, + and ++. (3) Ten bronchioles on each slice were randomly observed and respectively evaluated in respect of secretion within each bronchiole lumen and papillary hyperplasia of mucosal epithelial cells as four grades: -, +, ++ and +++. (4) The ratio of the mucosal wall area to the total area of bronchioles: Ten bronchioles from each animal were viewed with the same magnification in a fixed image distance with a multimedia color pathological image word analyses system (MPIAS-500) and the total areas and the lumina area of each bronchiole were depictured, from with the mucosal wall area of the bronchiole was calculated. Therefore, the degree of hyperplasia of the mucosal epithelial cells of the bronchiole was reflected by the mean value of the ratios of the mucosal wall areas to the total areas of the bronchioles.

### 3 Scoring of pathological changes and statistical treatment

(1) Pathological observations were performed on the chronic bronchitis model for the indices of inflammatory infiltration into bronchial mucosa, bronchial secretion, detachment of bronchial epithelial cells and hyperplasia of mucosal epithelial cells, with each index being scored semi-quantitatively as: "-" scored as 0; "+" as 1 point; "++" as 2 points; and "+++" as 3 points.
(2) The experimental results were pairwise compared among the means of the groups by *t*-test.

### 4. Test results:

### 4.1. Appearances and death

Appearances: In respect of air, activity and diet, the fumed animals in the model group were slightly inferior than those in the normal control group, and the animals in the test groups and in the GLKCNC group had no significant differences in comparison with those in the normal control group. During the course of the fuming and the fuming plus administration, none of the animals in the individual groups died.

### 4.2. Effect on the levels of interleukin-6

The results are shown in Table 10.

**Table 10 Effect on levels of interleukin-6 in mice suffering from fuming-induced chronic bronchitis**

| Group | Number of animals | Dosage | Measured value of interleukin-6 (*X̅*±SD) |
|---|---|---|---|
| | (n) | (g/kg) | (Pg/ml) |
| Control group | 15 | 25ml | 83.65±13.37 |
| Model group | 15 | 25ml | 122.27±16.57^{□□□} |
| GLKCNC group | 15 | 2 | 90.12±9.68*** |
| L-dosage group | 15 | 3 | 104.12±16.92** |
| M-dosage group | 15 | 6 | 99.07±14.36*** |
| H-dosage group | 15 | 12 | 105.68±9.98** |

| | | | |
|---|---|---|---|
| Note: ^{□□□}P<0.001, as compared to the control group; **P<0.01; ***P<0.001, as compared to the model group. | | | |

It can be seen from Table 10 that the measured value of interleukin-6 from the fumed model group is significantly higher than that of the control group (P<0.001), and the measured values of interleukin-6 from the fumed plus treatment groups are significantly less than that of the fumed model group (P<0.01, P<0.001). This indicates that the *in vivo* levels of interleukin-6 in mice of the medications groups are significantly decreased, which suggests inhibition of the inflammatory response and anti-infection defense of the mice.

### 4.3 Pathomorphological changes

Observation under optical microscope:
(1) The normal control group: the structure of alveoli was clear, the mucosal epithelia of bronchi and bronchioles of various divisions were intact, a few amount of secretion was present within trachea, hyperplasia of the mucosal epithelia of bronchi was inapparent, and there was a few inflammatory cells infiltrating around the bronchi and small vessels.
(2) The model group: the structure of the alveoli was passably clear, there was inflammatory cell infiltration into the periphery of the bronchi and bronchioles of various divisions to different extents, the mucosal epithelia of the bronchi could be observed to undergo degeneration, necrosis and detachment with various degrees, more secretion and hemorrhage were present within the lumen of the bronchi, and a part of mucosal epithelia of the bronchi underwent papillary hyperplasia.
(3) The fumed + GLKCNC group: the structure of the alveoli was passably clear, a mild inflammatory cell infiltrating could be observed at periphery of the bronchi and bronchioles of various divisions, the mucosal epithelia in the bronchi underwent significantly alleviated degeneration, necrosis and detachment as compared to those in the model group, secretion within the lumen of the bronchi was significantly reduced as compared to that in the model group, papillary hyperplasia of mucosal epithelia was significantly relieved as compared to that in the model group.
(4) The fumed + present medication group: the structure of the alveoli was passably clear, inflammation of different degrees in the bronchi and bronchioles of various divisions was significantly less than those in the model group, detachment of the mucosal epithelia was significantly alleviated, secretion within the lumen was significantly reduced as compared to the model group, and epithelial hyperplasia was also significantly reduced.

**Table 11 Effect of the present medication on inflammatory infiltration in bronchi (n=15)**

| Group | Dosage | Grade | | | Pathological score (*X̅*±SD) |
|---|---|---|---|---|---|
| | (g/kg) | + | ++ | +++ | |
| The normal control group | 25ml | 14 | 1 | 0 | 1.07±0.26 |
| Model group | 25ml | 5 | 8 | 2 | 1.80±0.68^{□□□} |
| The present medication | 3 | 12 | 2 | 1 | 1.27±0.59* |
| The present medication | 6 | 13 | 2 | 0 | 1.13±0.35** |
| The present medication | 12 | 14 | 1 | 0 | 1.07±0.26** |
| GLKCNC group | 2 | 11 | 4 | 0 | 1.27±0.46* |

Table 11 lammations epithelia th respect to the application and need your help. 5959595959595959595959595959595959595959595959595959595959595959595959 59 59 59 59 59 59 59 59 59 59 59 Note: ^{□□□}P<0.001, as compared to the control group; **P<0.05, ***P<0.001, as compared to the model group; "+" denotes that the area of bronchi and bronchioles of various divisions infiltrated by inflammatory cells in lung is less than 1/3 of the total area of a slice and inflammatory cells are less; "++" denotes that the area of bronchi and bronchioles of various divisions infiltrated by inflammatory cells in lung is ranging from 1/3 to 2/3 of the total area of a slice and inflammatory cells are more; "+++" denotes that the area of bronchi and bronchioles of various divisions infiltrated by inflammatory cell is above 2/3 of the total area of a slice and there are much more inflammatory cells.

It can be seen from Table 11 that inflammatory cells infiltration into bronchial mucosa in the model group increased which has significant difference as compared to the normal control group (P<0.01). Inflammatory cells infiltrations in the individual medication groups are significantly alleviated as compared to that in the normal control group and have significant differences (P<0.05, P<0.01).

**Table 12 Effect of the present medication on detachment of mucosal epithelia in bronchi (n=15)**

| Group | Dosage | Grade | | | Pathological score (*X̅*±SD) |
|---|---|---|---|---|---|
| | (g/kg) | - | + | ++ | |
| Normal control group | 25ml | 12 | 3 | 0 | 0.20±0.41 |
| Model group | 25ml | 1 | 10 | 4 | 1.20±0.56^{□□□} |
| The present medication | 3 | 9 | 4 | 2 | 0.53±0.74* |
| The present medication | 6 | 9 | 6 | 0 | 0.40±0.51*** |
| The present medication | 12 | 8 | 6 | 1 | 0.53±0.64** |
| GLKCNC group | 2 | 5 | 10 | 0 | 0.67±0.49* |

| | | | | | |
|---|---|---|---|---|---|
| Note: ^{□□□}P<0.001, as compared to the normal control group; **P<0.05, **P<0.01, ***P<0.001, as compared to the model group; "-" denotes normal state; "+" denotes that less than 1/3 of bronchial mucosal epithelia are detached in bronchial lumen and less than half of bronchi in total are found the detachment; "++" denotes that more than 1/3 bronchial mucosal epithelia are detached in bronchial lumen and more than half of bronchi in total are found the detachment. | | | | | |

It can be seen from Table 12 that the detachment of bronchial epithelial cells in the model group is significantly severer than that in the normal control group (P<0.001); the detachments of bronchial epithelial cells in the individual medication groups are significantly alleviated as compared to the model group (P<0.001, P<0.01, P<0.05).

**Table 13 Effect of the present medication on secretion in bronchiole lumen (n=15)**

| Group | Dosage | Grade | | | | Pathological score |
|---|---|---|---|---|---|---|
| | (g/kg) | - | + | ++ | +++ | (*X̅*±SD) |
| Normal control group | 25ml | 61 | 25 | 19 | 45 | 1.32±0.57 |
| Model group | 25ml | 20 | 49 | 9 | 72 | 1.89±0.39^{□□} |
| The present medication | 3 | 60 | 32 | 14 | 44 | 1.28±0.33*** |
| The present medication | 6 | 70 | 24 | 10 | 46 | 1.21±0.51*** |
| The present medication | 12 | 64 | 28 | 16 | 42 | 1.24±0.41*** |
| GLKCNC group | 2 | 67 | 37 | 12 | 34 | 1.09±0.53*** |

| | | | | | | |
|---|---|---|---|---|---|---|
| Note: ^{□□}P<0.01, as compared to the normal control group; ***P<0.001, as compared to the model group; "-" denotes normal state; "+" denotes that the volume of secretion in a bronchial lumen takes up less than 1/3 of the volume of the bronchial lumen; "++" denotes that the volume of secretion in a bronchiole lumen takes up 1/3 to 2/3 of the volume of the bronchial lumen; "+++" denotes that the volume of secretion in a bronchiole lumen takes up more than 2/3 of the volume of the bronchial lumen. | | | | | | |

It can be seen from Table 13 that the amount of the secretion and of hemorrhage within the bronchial lumen in the model group are larger than those in the normal control group with significant difference (P<0.001); and as compared to the model group, the amounts of the secretion within the bronchial lumen in the individual dose groups of the present medication are significantly reduced with significant difference (P<0.001, P<0.01, P<0.05).

**Table 14 Effect of the present medication on papillary hyperplasia of mucosal epithelia in bronchioles (n=15)**

| Group | Dosage | Grade | | | | Pathological score (*X̅*±SD) |
|---|---|---|---|---|---|---|
| | (g/kg) | - | + | ++ | +++ | |
| Normal control group | 25ml | 140 | 10 | 0 | 0 | 0.07±0.10 |
| Model group | 25ml | 74 | 60 | 14 | 2 | 0.63±0.32^{□□□} |
| The present medication | 3 | 116 | 26 | 8 | 0 | 0.28±0.18** |
| The present medication | 6 | 134 | 16 | 0 | 0 | 0.11±0.11*** |
| The present medication | 12 | 123 | 25 | 2 | 0 | 0.19±0.15*** |
| GLKCNC group | 2 | 124 | 26 | 0 | 0 | 0.17±0.16*** |

| | | | | | | |
|---|---|---|---|---|---|---|
| Note: ^{□□□}P<0.001, as compared to the normal control group; **P<0.01, ***P<0.001, as compared to the model group; "-" denotes normal state; "+" denotes that the area of papillary hyperplasia of mucosal epithelia in a bronchia is less than 1/3 of the total area of the bronchial with low extent; "++" denotes that the area of papillary hyperplasia of the mucosal epithelia in the bronchia amounts to between 1/3 and 2/3 of the total area of the bronchial lumen with higher extent; denotes that the volume of the secretion within the bronchiole lumen amounts to between 1/3 and 2/3 of the total area of the bronchial lumen; "+++" denotes that the area of papillary hyperplasia of the mucosal epithelia in the bronchia amounts to 2/3 above of the total area of the bronchial lumen with much higher extent. | | | | | | |

It can be seen from Table 14 that the papillary hyperplasia of mucosal epithelia of the bronchia in the model group are higher than that in the normal control group with significant difference (P<0.001); and as compared to the model group, the papillary hyperplasia of mucosal epithelia of the bronchia in the individual present medication groups is significantly lower than that in the model group (P<0.01, P<0.001).

**Table 15 Effect of the present medication on ratio of wall area/total area of bronchioles (n=15)**

| Group | Dosage | Treatment + fumed (*X̅*±SD) | | |
|---|---|---|---|---|
| | (g/kg) | Total area of bronchioles (µm²) | Area of bronchiolar lumen (µm²) | Wall area/total area of bronchiolar mucosa (µm²) |
| Control group | 25ml | 25949.0±13661.8 | 13661.8±4250.6 | 0.481±0.063 |
| Model group | 25ml | 38135.2±12560.8 | 11311.0±5776.4 | 0.715±0.055^{□□□} |
| The present medication | 3 | 28092.1±7694.7 | 13924.1±4017.3 | 0.517±0.056** |
| The present medication | 6 | 28993.8±9738.5 | 12680.5±3926.5 | 0.554±0.090*** |
| The present medication | 12 | 38553.5±13752.5 | 18559.2±5799.7 | 0.513±0.104*** |
| GLKCNC group | 2 | 31143.4±5667.7 | 14963.2±4993.2 | 0.5332±0.107*** |

| | | | | |
|---|---|---|---|---|
| Note: wall area of bronchiolar mucosa = total area of bronchiolar mucosa - area of bronchiolar lumen; ^{□□□}P<0.001, as compared to the control group; ***P<0.001, as compared to the model group. | | | | |

It can be seen from Table 15 that the wall area of bronchiolar mucosa in the model group is larger than that in the control group with significant difference (P<0.001); and the wall area of bronchiolar mucosa in the individual medication groups is significantly less than that in the model group (P<0.001). This indicates that all of the dosages of the present medication in the individual dose groups can alleviate hyperplasia of bronchiolar epithelial cells, enlarge the area of bronchiolar lumen, and increase the ventilation volume.

### 5 Conclusion:

A mouse model of chronic bronchitis was established with cigarettes as the single stimulus. Under the same condition as in the model group, the mice in the test groups of the present medication were intragastrically administered. The experimental results show that the air, activity and diet of the mice in the test groups of the present medication were superior to those in the model group, and similar to those in the control group. The present medication significantly decreased the *in vivo* level of interleukin-6 in mice, which inhibited the inflammatory response and anti-infection defense of the mice's bodies. From the microscopic level, the extent of inflammation on the bronchial mucosa, the amount of the bronchial secretion, and the detachment and hyperplasia of bronchial epithelial cells in the mice of the test groups were alleviated to different extent than those in the model group, with significant differences.

It has been shown experimentally that the present medication can improve various pathological changes of chronic bronchitis induced by passive smoking in mice, suggesting good effect of the present medication in treatment of the chronic bronchitis.

### Test Example 6 Investigation on toxicology of the present medication

### 1. Acut toxicity test (maximum dose experiment)

The experiment of acute toxicity of mice was performed with respect of the present medication. Since of the value of LD₅₀ was failed to be measured, a maximum dose experiment was performed instead. Mice were intragastrically administered at the maximum volume with the maximum concentration. The result indicates that when the mice were administered with a maximum dosage of 243 g crude drugs/kg which is 378 times of the clinical dosage (the clinical dosage is 45 g crude drugs/person/day, the assumed body weight of a person being 70kg), no adverse reaction or death was observed. The animals were autopsied at the end of the experiment and no abnormality of each organ was observed with naked eyes.

### 2. Long-term toxicity test

120 Wistar rats were used in the experiment, which were assigned into the control group and the test groups of the present medication with dosages of 8g, 16g, and 32g crude drugs/kg, which are 12.5, 25 and 50 times of the clinical dosage (45g crude drugs/person/day) respectively, and intragastricall administered for consecutive 24 weeks. The animals were observed for the influence of the present medication on various indices. The experimental results show that the dosages of the present medication have no apparent influence on body weight, feed consumption, peripheral hemogram, electrocardiogram, and organ index; PTs in the rats of the low dosage group in 12 weeks post administration as well as the high, medium and low dosages groups in 24 weeks post administration are lower than those in the control group, which is the statistical difference due to the low standard deviation of the measured values and is meaningless physiologically; and during the recovery period (medication withdrawal for 4 weeks), PT and APTT in the test groups are of no significant difference as compared to those in the control group. Among the measured values for the twelve indices of blood biochemistry, the GLU value of the low dosage group in 12 weeks post administration is higher than that of the control group (P<0.05) but still falls within the normal value range for the physiological index. AST, TP and CRE values in the low dosage group as well as ALP and TP values in the medium dosage group in 12 weeks post administration are lower than those of the control group (P<0.05), but these indices still fall within respective normal value ranges. After administration for 24 weeks, AST, TP and CRE values of the medium and high dosages groups are lower than those of the control group (P<0.05, P<0.01), and ALB and BUN values of the high dosage group are lower than those of the control group (P<0.05, P<0.01), but these indices still fall within the respective normal value ranges. During the recovery period, BiLi value of the low dosage group is higher than that of the control group (P<0.05), and CRE and BUN values of the low dosage group is lower than those of the control group (P<0.05, P<0.01), and these indices still fall within the respective normal value ranges. Other indices are of no significant difference in comparison with the control group. In the pathological examination, no apparent pathological changes caused by the present medication in the organs are found.

### 3. Summary

In the acute toxicity test (the maximum dose experiment) and the long-term toxicity test, the various indices in the test groups do not show significant differences in comparison with the control group. In the pathological examination, no apparent pathological changes caused by the present medication in the organs are found.

In view of the above, the present medication has effects of checking cough, dispelling phlegm, relieving dyspnea and resisting inflammation, and has a good therapeutic efficacy on bronchitis, particularly has an excellent efficacy on chronic bronchitis.

## Claims

1. A pharmaceutical composition for use in treating bronchitis, **characterized in that**, said pharmaceutical composition is made from the medicinal materials in the following parts by weight:
| | | | |
|---|---|---|---|
| CITRI RETICULATAE PERICARPIUM 94 - 156 | PINELLIAE RHIZOMA 94 - 156 | ASTERIS RADIX ET RHIZOMA 94 - 156 | FARFARAE FLOS 94 - 156 |
| PORIA 94-156 | ATRACTYLODIS MACROCEPHALAE RHIZOMA 94-156 | ARMENIACAE SEMEN AMARUM 78-130 | PERILLAE FRUCTUS 78 - 130 |
| SINAPIS SEMEN 78-130 | RAPHANI SEMEN 78-130 | CINNAMOMI RAMULUS 46 - 78 | SCUTELLARIAE RADIX 94-156 |
| FORSYTHIAE FRUCTUS 94 - 156 | HOUTTUYNIAE HERBA 225 - 391 | and GLYCYRRHIZAE RADIX ET RHIZOMA 31 - 53. | |

2. The pharmaceutical composition for use in treating bronchitis according to Claim 1, **characterized in that**, said pharmaceutical composition is made from the medicinal materials in the following parts by weight:
| | | | |
|---|---|---|---|
| CITRI RETICULATAE PERICARPIUM 94 | PINELLIAE RHIZOMA 156 | ASTERIS RADIX ET RHIZOMA 94 | FARFARAE FLOS 156 |
| PORIA 94 | ATRACTYLODIS MACROCEPHALAE RHIZOMA 156 | ARMENIACAE SEMEN AMARUM 78 | PERILLAE FRUCTUS 130 |
| SINAPIS SEMEN 78 | RAPHANI SEMEN 130 | CINNAMOMI RAMULUS 46 | SCUTELLARIAE RADIX 94 |
| FORSYTHIAE FRUCTUS 156 | HOUTTUYNIAE HERBA 391 | and GLYCYRRHIZAE RADIX ET RHIZOMA 31. | |

3. The pharmaceutical composition for use in treating bronchitis according to Claim 1, **characterized in that**, said pharmaceutical composition is made from the medicinal materials in the following parts by weight:
| | | | |
|---|---|---|---|
| CITRI RETICULATAE PERICARPIUM 156 | PINELLIAE RHIZOMA 94 | ASTERIS RADIX ET RHIZOMA 156 | FARFARAE FLOS 94 |
| PORIA 156 | ATRACTYLODIS MACROCEPHALAE RHIZOMA 94 | ARMENIACAE SEMEN AMARUM 130 | PERILLAE FRUCTUS 78 |
| SINAPIS SEMEN 130 | RAPHANI SEMEN 78 | CINNAMOMI RAMULUS 78 | SCUTELLARIAE RADIX 156 |
| FORSYTHIAE FRUCTUS 94 | HOUTTUYNIAE HERBA 225 | and GLYCYRRHIZAE RADIX ET RHIZOMA 53. | |

4. The pharmaceutical composition for use in treating bronchitis according to Claim 1, **characterized in that**, said pharmaceutical composition is made from the medicinal materials in the following parts by weight:
| | | | |
|---|---|---|---|
| CITRI RETICULATAE PERICARPIUM 125 | PINELLIAE RHIZOMA 125 | ASTERIS RADIX ET RHIZOMA 125 | FARFARAE FLOS 125 |
| PORIA 125 | ATRACTYLODIS MACROCEPHALAE RHIZOMA 125 | ARMENIACAE SEMEN AMARUM 104 | PERILLAE FRUCTUS 104 |
| SINAPIS SEMEN 104 | RAPHANI SEMEN 104 | CINNAMOMI RAMULUS 62 | SCUTELLARIAE RADIX 125 |
| FORSYTHIAE FRUCTUS 125 | HOUTTUYNIAE HERBA 313 | and GLYCYRRHIZAE RADIX ET RHIZOMA 42. | |

5. The pharmaceutical composition for use in treating bronchitis according to Claim 1, **characterized in that**, said pharmaceutical composition is made from the medicinal materials in the following parts by weight:
| | | | |
|---|---|---|---|
| CITRI RETICULATAE PERICARPIUM 98 | PINELLIAE RHIZOMA 98 | ASTERIS RADIX ET RHIZOMA 104 | FARFARAE FLOS 104 |
| PORIA 144 | ATRACTYLODIS MACROCEPHALAE RHIZOMA 148 | ARMENIACAE SEMEN AMARUM 85 | PERILLAE FRUCTUS 85 |
| SINAPIS SEMEN 125 | RAPHANI SEMEN 128 | CINNAMOMI RAMULUS 75 | SCUTELLARIAE RADIX 151 |
| FORSYTHIAE FRUCTUS 149 | HOUTTUYNIAE HERBA 377 | and GLYCYRRHIZAE RADIX ET RHIZOMA 49. | |

6. The pharmaceutical composition for use in bronchitis according to Claim 1, **characterized in that**, said pharmaceutical composition is made from the medicinal materials in the following parts by weight:
| | | | |
|---|---|---|---|
| CITRI RETICULATAE | PINELLIAE RHIZOMA | ASTERIS RADIX ET | FARFARAE FLOS 140 |
| PERICARPIUM 147 | 152 | RHIZOMA 149 | |
| | | | |
| PORIA 105 | ATRACTYLODIS | ARMENIACAE SEMEN | PERILLAE FRUCTUS 81 |
| | MACROCEPHALAE | AMARUM 81 | |
| | RHIZOMA 105 | | |
| | | | |
| SINAPIS SEMEN 125 | RAPHANI SEMEN 125 | CINNAMOMI RAMULUS | SCUTELLARIAE RADIX |
| | | 74 | 149 |
| | | | |
| FORSYTHIAE FRUCTUS | HOUTTUYNIAE HERBA | and GLYCYRRHIZAE | |
| 105 | 276 | RADIX ET RHIZOMA 45. | |

7. The pharmaceutical composition for use according to any one of Claims 1-6, **characterized in that**, PINELLIAE RHIZOMA is PINELLIAE RHIZOMA PRAEPARATUM CUM ALUMINE, ATRACTYLODIS MACROCEPHALAE RHIZOMA is RHIZOMA ATRACTYLODIS MACROCEPHALAE TOSTUM CUM MELLE ET FURFURE, ARMENIACAE SEMEN AMARUM is ARMENIACAE SEMEN AMARUM TOSTUM, and SCUTELLARIAE RADIX is in a form of slices.

8. The pharmaceutical composition for use according to any one of Claims 1-7, **characterized in that**, the pharmaceutical composition is prepared in steps of:
(1) weighing selected clean SCUTELLARIAE RADIX according to the part set forth in the prescription and pulverizing the same into fine powder for later use;
(2) weighing selected clean CITRI RETICULATAE PERICARPIUM and CINNAMOMI RAMULUS according to the parts set forth in the prescription, adding 8-12 folds by v/w (ml/g) of water based on the weight of the medicinal materials, and subjecting the mixture to distillation for 3-7 h to extract essential oils, collecting the essential oils for later use;
(3) collecting the aqueous solution obtained after the distillation in step (2) in a container, extracting the herbal residue from step (2) together with ATRACTYLODIS MACROCEPHALAE RHIZOMA, PORIA, GLYCYRRHIZAE RADIX ET RHIZOMA, FARFARAE FLOS and FORSYTHIAE FRUCTUS twice with water, each time for 1-3 hours, in which an amount of water is 7-11 times by v/w (ml/g) of the weight of the medicinal materials for the first time and 5-9 times by v/w (ml/g) for the second time; then, pooling the aqueous extracts and filtrating the mixture, combining the filtrate with the above said aqueous solution obtained after the distillation, and concentrating to a clear paste with the relative density of 1.15-1.20 thermally measured at 60 °C for later ues;
(4) weighing selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the parts set forth in the prescription, extracting the same twice in 5-7 folds by v/w (ml/g) of 40-70% ethanol solution based on the weight of the medicinal materials, the first time for 1-3 hours and the second time for 1-2 hours; pooling the extracts and filtrating the mixture; recovering ethanol of the filtrate, concentrating the residue to a clear paste with the relative density of 1.15-1.20 thermally measured at 60 °C, and combining the clear paste with the clear paste obtained in step (3) for later use;
the fine powder obtained in step (1), the essential oils obtained in step (2) and the combined clear pastes obtained in step (4) constitute the active ingredients of the pharmaceutical composition.

9. The pharmaceutical composition for use according to any one of Claims 1-8, **characterized in that**, the pharmaceutical composition is in a dosage form of hard capsule, tablet, powder, oral liquid, soft capsule, pill, tincture, syrup, suppository, gel, spray, or injection.

10. The pharmaceutical composition for use according to Claims 9, **characterized in that**, a tablet of the pharmaceutical composition is formulated in steps of:
(1) weighing selected clean SCUTELLARIAE RADIX according to the part set forth in the prescription and pulverizing the same into fine powder for later use SCUTELLARIAE RADIX;
(2) weighing selected clean CITRI RETICULATAE PERICARPIUM and CINNAMOMI RAMULUS according to the parts set forth in the prescription, adding 8-12 folds by v/w (ml/g) of water based on the weight of the medicinal materials, subjecting the mixture to distillation for 4-6 h to extract essential oils, and collecting the essential oils for later use;
(3) collecting the aqueous solution obtained after the distillation in step (2) in a container, extracting the herbal residue from step (2) together with ATRACTYLODIS MACROCEPHALAE RHIZOMA, PORIA, GLYCYRRHIZAE RADIX ET RHIZOMA, FARFARAE FLOS and FORSYTHIAE FRUCTUS twice with water, each time for 1-3 hours, in which an amount of water is 7-11 times by v/w (ml/g) of the weight of the medicinal materials for the first time and 5-9 times by v/w (ml/g) for the second time; then, pooling the aqueous extracts and filtrating the mixture, combining the filtrate with the above said aqueous solution obtained after the distillation, and concentrating to a clear paste with the relative density of 1.15-1.20 thermally measured at 60 °C for later ues;
(4) weighing selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the parts set forth in the prescription, extracting the same twice with 5-7 folds by v/w (ml/g) of 40-70% ethanol solution based on the weight of the medicinal materials, the first time for 1-3 hours and the second time for 1-2 hours; pooling the extracts and filtrating the mixture; recovering ethanol of the filtrate, concentrating the residue to a clear paste with the relative density of 1.15-1.20 thermally measured at 60 °C, and combining the clear paste with the clear paste obtained in step (3) for later use;
(5) granulating the combined clear pastes obtained in step (4) with the fine powder obtained in step (1) as a fundamental material, and finishing the obtained granules for later use;
(6) providing the materials in parts by weight for tableting as follows:
granules obtained in step (5) 360 - 600 colloidal silicon dioxide 1.8 - 3.2
magnesium stearate 1.8 - 3.2
(7) tableting by a conventional formulation process to obtain the tablet of the pharmaceutical composition.

11. The pharmaceutical composition for use according to Claim 10 ,**characterized in that**, the tablet of the pharmaceutical composition is formulated in steps of:
(1) weighing selected clean SCUTELLARIAE RADIX according to the part set forth in the prescription and pulverizing the same into fine powder for later use SCUTELLARIAE RADIX;
(2) weighing selected clean CITRI RETICULATAE PERICARPIUM and CINNAMOMI RAMULUS according to the parts set forth in the prescription, adding 9 folds by v/w (ml/g) of water based on the weight of the medicinal materials, subjecting the mixture to distillation for 5 h to extract essential oils, and collecting the essential oils for later use;
(3) collecting the aqueous solution obtained after the distillation in step (2) in a container, extracting the herbal residue from step (2) together with ATRACTYLODIS MACROCEPHALAE RHIZOMA, PORIA, GLYCYRRHIZAE RADIX ET RHIZOMA, FARFARAE FLOS and FORSYTHIAE FRUCTUS twice with water, each time for 4 hours, in which an amount of water is 10 times by v/w (ml/g) of the weight of the medicinal materials for the first time and 7 times by v/w (ml/g) for the second time; then, pooling the aqueous extracts and filtrating the mixture, combining the filtrate with the above said aqueous solution obtained after the distillation, and concentrating to a clear paste with the relative density of 1.17 thermally measured at 60 °C for later ues;
(4) weighing selected clean PINELLIAE RHIZOMA, PERILLAE FRUCTUS, SINAPIS SEMEN, RAPHANI SEMEN, ASTERIS RADIX ET RHIZOMA, HOUTTUYNIAE HERBA, and ARMENIACAE SEMEN AMARUM according to the parts set forth in the prescription, extracting the same twice with 6 folds by v/w (ml/g) of 60% ethanol solution based on the weight of the medicinal materials, the first time for 2 hours and the second time for 1 hour; pooling the extracts and filtrating the mixture; recovering ethanol of the filtrate, concentrating the residue to a clear paste with the relative density of 1.17 thermally measured at 60 °C, and combining the clear paste with the clear paste obtained in step (3) for later use;
(5) granulating the combined clear pastes obtained in step (4) with the fine powder obtained in step (1) as a fundamental material, and finishing the obtained granules for later use;
(6) providing the materials in parts by weight for tableting as follows:
granules obtained in step (5) 470 colloidal silicon dioxide 2.4
magnesium stearate 2.4;
(7) adding the essential oils obtained in step (2) to the colloidal silicon dioxide and tableting by a convetional formulation process to obtain the tablet of the pharmaceutical composition.

12. The pharmaceutical composition for use in treating bronchitis according to any one of Claims 1-11, **characterized in that**, said bronchitis is chronic bronchitis.

## Patentansprüche

1. Pharmazeutische Zusammensetzung zur Verwendung bei der Behandlung von Bronchitis, **dadurch gekennzeichnet, dass** die pharmazeutische Zusammensetzung aus den medizinischen Materialen mit den folgenden Gewichtsanteilen hergestellt ist:
| | | | |
|---|---|---|---|
| CITRI RETICULATAE PERICARPIUM 94-156 | PINELLIAE RHIZOMA 94 - 156 | ASTERIS RADIX ET RHIZOMA 94-156 | FARFARAE FLOS 94 - 156 |
| PORIA 94 - 156 | ATRACTYLODIS MACROCEPHALAE RHIZOMA 94-156 | ARMENIACAE SAMEN AMARUM 78 - 130 | PERILLAE FRUCTUS 78-130 |
| SINAPIS SEMEN 78 - 130 | RAPHANI SEMEN 78 - 130 | CINNAMOMI RAMULUS 46-78 | SCUTELLARIAE RADIX 94-156 |
| FORSYTHIAE FRUCTUS 94-156 | HOUTTUYNIAE HERBA 225 - 391 | und GLYCYRRHIZAE RADIX ET RHIZOMA 31 - 53. | |

2. Pharmazeutische Zusammensetzung zur Verwendung bei der Behandlung von Bronchitis nach Anspruch 1, **dadurch gekennzeichnet, dass** die pharmazeutische Zusammensetzung aus den medizinischen Materialen mit den folgenden Gewichtsanteilen hergestellt ist:
| | | | |
|---|---|---|---|
| CITRI RETICULATAE PERICARPIUM 94 | PINELLIAE RHIZOMA 156 | ASTERIS RADIX ET RHIZOMA 94 | FARFARAE FLOS 156 |
| PORIA 94 | ATRACTYLODIS MACROCEPHALAE RHIZOMA 156 | ARMENIACAE SAMEN AMARUM 78 | PERILLAE FRUCTUS 130 |
| SINAPIS SEMEN 78 | RAPHANI SEMEN 130 | CINNAMOMI RAMULUS 46 | SCUTELLARIAE RADIX 94 |
| FORSYTHIAE FRUCTUS 156 | HOUTTUYNIAE HERBA 391 | und GLYCYRRHIZAE RADIX ET RHIZOMA 31. | |

3. Pharmazeutische Zusammensetzung zur Verwendung bei der Behandlung von Bronchitis nach Anspruch 1, **dadurch gekennzeichnet, dass** die pharmazeutische Zusammensetzung aus den medizinischen Materialen mit den folgenden Gewichtsanteilen hergestellt ist:
| | | | |
|---|---|---|---|
| CITRI RETICULATAE PERICARPIUM 156 | PINELLIAE RHIZOMA 94 | ASTERIS RADIX ET RHIZOMA 156 | FARFARAE FLOS 94 |
| PORIA 156 | ATRACTYLODIS MACROCEPHALAE RHIZOMA 94 | ARMENIACAE SAMEN AMARUM 130 | PERILLAE FRUCTUS 78 |
| SINAPIS SEMEN 130 | RAPHANI SEMEN 78 | CINNAMOMI RAMULUS 78 | SCUTELLARIAE RADIX 156 |
| FORSYTHIAE FRUCTUS 94 | HOUTTUYNIAE HERBA 225 | und GLYCYRRHIZAE RADIX ET RHIZOMA 53. | |

4. Pharmazeutische Zusammensetzung zur Verwendung bei der Behandlung von Bronchitis nach Anspruch 1, **dadurch gekennzeichnet, dass** die pharmazeutische Zusammensetzung aus den medizinischen Materialen mit den folgenden Gewichtsanteilen hergestellt ist:
| | | | |
|---|---|---|---|
| CITRI RETICULATAE PERICARPIUM 125 | PINELLIAE RHIZOMA 125 | ASTERIS RADIX ET RHIZOMA 125 | FARFARAE FLOS 125 |
| PORIA 125 | ATRACTYLODIS MACROCEPHALAE RHIZOMA 125 | ARMENIACAE SAMEN AMARUM 104 | PERILLAE FRUCTUS 104 |
| SINAPIS SEMEN 104 | RAPHANI SEMEN 104 | CINNAMOMI RAMULUS 62 | SCUTELLARIAE RADIX 125 |
| FORSYTHIAE FRUCTUS 125 | HOUTTUYNIAE HERBA 313 | und GLYCYRRHIZAE RADIX ET RHIZOMA 42. | |

5. Pharmazeutische Zusammensetzung zur Verwendung bei der Behandlung von Bronchitis nach Anspruch 1, **dadurch gekennzeichnet, dass** die pharmazeutische Zusammensetzung aus den medizinischen Materialen mit den folgenden Gewichtsanteilen hergestellt ist:
| | | | |
|---|---|---|---|
| CITRI RETICULATAE PERICARPIUM 98 | PINELLIAE RHIZOMA 98 | ASTERIS RADIX ET RHIZOMA 104 | FARFARAE FLOS 104 |
| PORIA 144 | ATRACTYLODIS MACROCEPHALAE RHIZOMA 148 | ARMENIACAE SAMEN AMARUM 85 | PERILLAE FRUCTUS 85 |
| SINAPIS SEMEN 125 | RAPHANI SEMEN 128 | CINNAMOMI RAMULUS 75 | SCUTELLARIAE RADIX 151 |
| FORSYTHIAE FRUCTUS 149 | HOUTTUYNIAE HERBA 377 | und GLYCYRRHIZAE RADIX ET RHIZOMA 49. | |

6. Pharmazeutische Zusammensetzung zur Verwendung bei der Behandlung von Bronchitis nach Anspruch 1, **dadurch gekennzeichnet, dass** die pharmazeutische Zusammensetzung aus den medizinischen Materialen mit den folgenden Gewichtsanteilen hergestellt ist:
| | | | |
|---|---|---|---|
| CITRI RETICULATAE PERICARPIUM 147 | PINELLIAE RHIZOMA 152 | ASTERIS RADIX ET RHIZOMA 149 | FARFARAE FLOS 140 |
| PORIA 105 | ATRACTYLODIS MACROCEPHALAE RHIZOMA 105 | ARMENIACAE SAMEN AMARUM 81 | PERILLAE FRUCTUS 81 |
| SINAPIS SEMEN 125 | RAPHANI SEMEN 125 | CINNAMOMI RAMULUS 74 | SCUTELLARIAE RADIX 149 |
| FORSYTHIAE FRUCTUS 105 | HOUTTUYNIAE HERBA 276 | und GLYCYRRHIZAE RADIX ET RHIZOMA 45. | |

7. Pharmazeutische Zusammensetzung zur Verwendung nach einem der Ansprüche 1 - 6, **dadurch gekennzeichnet, dass**: PINELLIAE RHIZOMA ist PINELLIAE RHIZOMA PRAEPARATUM CUM ALUMINE, ATRACTYLODIS MACROCEPHALAE RHIZOMA ist RHIZOMA ATRACTYLODIS MACROCEPHALAE TOSTUM CUM MELLE ET FURFURE, ARMENIACAE SAMEN AMARUM ist ARMENIACAE SAMEN AMARUM TOSTUM und SCUTELLARIAE RADIX ist in der Form von Scheiben.

8. Pharmazeutische Zusammensetzung zur Verwendung nach einem der Ansprüche 1 - 7, **dadurch gekennzeichnet, dass** die pharmazeutische Zusammensetzung in den Schritten hergestellt wird:
(1) Wiegen von ausgewähltem, sauberem SCUTELLARIAE RADIX entsprechend dem in der Beschreibung dargelegten Teil und Pulverisieren desselben zu einem feinen Pulver für die spätere Verwendung;
(2) Wiegen von ausgewähltem, sauberem CITRI RETICULATAE PERICARPIUM und CINNAMOMI RAMULUS entsprechend den in der Beschreibung dargelegten Teilen, Zugeben der 8 - 12-fachen Menge v/w (ml/g) an Wasser, basierend auf dem Gewicht der medizinischen Materialien, Unterziehen der Mischung einer Destillation für 3 - 7 h, um die essentiellen Öle zu extrahieren, und Sammeln der essentiellen Öle für die spätere Verwendung;
(3) Sammeln der wässerigen Lösung, die nach der Destillation in dem Schritt (2) erhalten wird, in einem Behälter, zweimaliges Extrahieren des pflanzlichen Rests aus dem Schritt (2) zusammen mit ATRACTYLODIS MACROCEPHALAE RHIZOMA, PORIA, GLYCYRRHIZAE RADIX ET RHIZOMA, FARFARAE FLOS und FORSYTHIAE FRUCTUS mit Wasser, jedes Mal für 1 - 3 Stunden, wobei beim ersten Mal eine Wassermenge 7 - 11-mal v/w (ml/g) das Gewicht von den medizinischen Materialien ist und beim zweiten Mal 5 - 9-mal v/w (ml/g); dann Vereinen der wässerigen Extrakte und Filtrieren der Mischung, Kombinieren des Filtrats mit der oben genannten wässerigen Lösung, die nach der Destillation erhalten wird, und Konzentrieren zu einer klaren Paste mit der relativen Dichte von 1,15 - 1,20, thermisch bei 60 °C gemessen, für die spätere Verwendung;
(4) Wiegen von ausgewähltem, sauberem PINELLIAE RHIZOMA, PERILLAE FRUCTUS, SINAPIS SAMEN, RAPHANI SAMEN, ASTERIS RADIX ET RHIZOMA, HOUTTUYNIAE HERBA und ARMENIACAE SAMEN AMARUM entsprechend den in der Beschreibung dargelegten Teilen, zweimaliges Extrahieren derselben in einer 5 - 7-fachen v/w (ml/g) 40 - 70 % Ethanollösung, basierend auf dem Gewicht der medizinischen Materialien, das erste Mal für 1 - 3 Stunden und das zweite Mal für 1 - 2 Stunden; Vereinen der Extrakte und Filtrieren der Mischung; Rückgewinnen des Ethanols aus dem Filtrat, Konzentrieren des Rests zu einer klaren Paste mit der relativen Dichte von 1,15 - 1,20, thermisch bei 60 °C gemessen, und Kombinieren der klaren Paste mit der klaren Paste, die im Schritt (3) erhalten wird, für die spätere Verwendung;
wobei das feine Pulver, das im Schritt (1) erhalten wird, die essentiellen Öle, die im Schritt (2) erhalten werden, und die kombinierten klaren Plasten, die im Schritt (4) erhalten werden, die aktiven Bestandteile der pharmazeutischen Zusammensetzung bilden.

9. Pharmazeutische Zusammensetzung zur Verwendung nach einem der Ansprüche 1 - 8, **dadurch gekennzeichnet, dass** die pharmazeutische Zusammensetzung in einer Dosierungsform von einer Hartkapsel, Tablette, Pulver, Oralflüssigkeit, Weichkapsel, Pille, Tinktur, Sirup, Suppositorium, Gel, Spray oder Injektion ist.

10. Pharmazeutische Zusammensetzung zur Verwendung nach Anspruch 9, **dadurch gekennzeichnet, dass** eine Tablette der pharmazeutischen Zusammensetzung in den Schritten hergestellt wird:
(1) Wiegen von ausgewähltem, sauberem SCUTELLARIAE RADIX entsprechend dem in der Beschreibung dargelegten Teil und Pulverisieren desselben zu einem feinen Pulver für die spätere Verwendung von SCUTELLARIAE RADIX;
(2) Wiegen von ausgewähltem, sauberem CITRI RETICULATAE PERICARPIUM und CINNAMOMI RAMULUS entsprechend den in der Beschreibung dargelegten Teilen, Zugeben der 8 - 12-fachen Menge v/w (ml/g) an Wasser, basierend auf dem Gewicht der medizinischen Materialien, Unterziehen der Mischung einer Destillation für 4 - 6 h, um die essentiellen Öle zu extrahieren, und Sammeln der essentiellen Öle für die spätere Verwendung;
(3) Sammeln der wässerigen Lösung, die nach der Destillation in dem Schritt (2) erhalten wird, in einem Behälter, zweimaliges Extrahieren des pflanzlichen Rests aus dem Schritt (2) zusammen mit ATRACTYLODIS MACROCEPHALAE RHIZOMA, PORIA, GLYCYRRHIZAE RADIX ET RHIZOMA, FARFARAE FLOS und FORSYTHIAE FRUCTUS mit Wasser, jedes Mal für 1 - 3 Stunden, wobei beim ersten Mal eine Wassermenge 7 - 11-mal v/w (ml/g) das Gewicht von den medizinischen Materialien ist und beim zweiten Mal 5 - 9-mal v/w (ml/g); dann Vereinen der wässerigen Extrakte und Filtrieren der Mischung, Kombinieren des Filtrats mit der oben genannten wässerigen Lösung, die nach der Destillation erhalten wird, und Konzentrieren zu einer klaren Paste mit der relativen Dichte von 1,15 - 1,20, thermisch bei 60 °C gemessen, für die spätere Verwendung;
(4) Wiegen von ausgewähltem, sauberem PINELLIAE RHIZOMA, PERILLAE FRUCTUS, SINAPIS SAMEN, RAPHANI SAMEN, ASTERIS RADIX ET RHIZOMA, HOUTTUYNIAE HERBA und ARMENIACAE SAMEN AMARUM entsprechend den in der Beschreibung dargelegten Teilen, zweimaliges Extrahieren derselben in einer 5 - 7-fachen v/w (ml/g) 40 - 70 % Ethanollösung, basierend auf dem Gewicht der medizinischen Materialien, das erste Mal für 1 - 3 Stunden und das zweite Mal für 1 - 2 Stunden; Vereinen der Extrakte und Filtrieren der Mischung; Rückgewinnen des Ethanols aus dem Filtrat, Konzentrieren des Rests zu einer klaren Paste mit der relativen Dichte von 1,15 - 1,20, thermisch bei 60 °C gemessen, und Kombinieren der klaren Paste mit der klaren Paste, die im Schritt (3) erhalten wird, für die spätere Verwendung;
(5) Granulieren der kombinierten klaren Pasten, die im Schritt (4) erhalten werden, mit dem feinem Pulver, das in dem Schritt (1) erhalten wird, als ein Basismaterial und Endbearbeiten des erhaltenen Granulats für die spätere Verwendung;
(6) Bereitstellen der Materialien in Gewichtsanteilen zur Tablettierung und zwar wie folgt:
| | |
|---|---|
| Granulat, erhalten im Schritt (5) | 360 - 600 |
| Kolloidales Siliciumdioxid | 1,8 - 3,2 |
| Magnesiumstearat | 1,8 - 3,2 |
(7) Tablettieren mittels eines herkömmlichem Formulierungsverfahrens, um die Tablette der pharmazeutischen Zusammensetzung zu erhalten.

11. Pharmazeutische Zusammensetzung zur Verwendung nach Anspruch 10, **dadurch gekennzeichnet, dass** die Tablette der pharmazeutischen Zusammensetzung in den Schritten formuliert wird:
(1) Wiegen von ausgewähltem, sauberem SCUTELLARIAE RADIX entsprechend dem in der Beschreibung dargelegten Teil und Pulverisieren desselben zu einem feinen Pulver für die spätere Verwendung von SCUTELLARIAE RADIX;
(2) Wiegen von ausgewähltem, sauberem CITRI RETICULATAE PERICARPIUM und CINNAMOMI RAMULUS entsprechend den in der Beschreibung dargelegten Teilen, Zugeben der 9-fachen Menge v/w (ml/g) an Wasser, basierend auf dem Gewicht der medizinischen Materialien, Unterziehen der Mischung einer Destillation für 5 h, um die essentiellen Öle zu extrahieren, und Sammeln der essentiellen Öle für die spätere Verwendung;
(3) Sammeln der wässerigen Lösung, die nach der Destillation in dem Schritt (2) erhalten wird, in einem Behälter, zweimaliges Extrahieren des pflanzlichen Rests aus dem Schritt (2) zusammen mit ATRACTYLODIS MACROCEPHALAE RHIZOMA, PORIA, GLYCYRRHIZAE RADIX ET RHIZOMA, FARFARAE FLOS und FORSYTHIAE FRUCTUS mit Wasser, jedes Mal für 4 Stunden, wobei beim ersten Mal eine Wassermenge 10-mal v/w (ml/g) das Gewicht von den medizinischen Materialien ist und beim zweiten Mal 7-mal v/w (ml/g); dann Vereinen der wässerigen Extrakte und Filtrieren der Mischung, Kombinieren des Filtrats mit der oben genannten wässerigen Lösung, die nach der Destillation erhalten wird, und Konzentrieren zu einer klaren Paste mit der relativen Dichte von 1,17, thermisch bei 60 °C gemessen, für die spätere Verwendung;
(4) Wiegen von ausgewähltem, sauberem PINELLIAE RHIZOMA, PERILLAE FRUCTUS, SINAPIS SAMEN, RAPHANI SAMEN, ASTERIS RADIX ET RHIZOMA, HOUTTUYNIAE HERBA und ARMENIACAE SAMEN AMARUM entsprechend den in der Beschreibung dargelegten Teilen, zweimaliges Extrahieren derselben in einer 6-fachen v/w (ml/g) 60 % Ethanollösung, basierend auf dem Gewicht der medizinischen Materialien, das erste Mal für 2 Stunden und das zweite Mal für 1 Stunde; Vereinen der Extrakte und Filtrieren der Mischung; Rückgewinnen des Ethanols aus dem Filtrat, Konzentrieren des Rests zu einer klaren Paste mit der relativen Dichte von 1,17, thermisch bei 60 °C gemessen, und Kombinieren der klaren Paste mit der klaren Paste, die im Schritt (3) erhalten wird, für die spätere Verwendung;
(5) Granulieren der kombinierten klaren Pasten, die im Schritt (4) erhalten werden, mit dem feinem Pulver, das in dem Schritt (1) erhalten wird, als ein Basismaterial und Endbearbeiten des erhaltenen Granulats für die spätere Verwendung;
(6) Bereitstellen der Materialien in Gewichtsanteilen zur Tablettierung und zwar wie folgt:
| | |
|---|---|
| Granulat, erhalten im Schritt (5) | 470 |
| Kolloidales Siliciumdioxid | 2,4 |
| Magnesiumstearat | 2,4 |
(7) Zugeben der essentiellen Öle, die im Schritt (2) erhalten werden, zu dem kolloidalen Siliciumoxid und Tablettieren mittels eines herkömmlichem Formulierungsverfahrens, um die Tablette der pharmazeutischen Zusammensetzung zu erhalten.

12. Pharmazeutische Zusammensetzung zur Verwendung bei der Behandlung von Bronchitis nach einem der Ansprüche 1 - 11, **dadurch gekennzeichnet, dass** die Bronchitis eine chronische Bronchitis ist.

## Revendications

1. Composition pharmaceutique pour une utilisation dans le traitement de la bronchite, **caractérisée en ce que** ladite composition pharmaceutique est préparée à partir des produits à visée médicale dans les parties en poids suivantes :
| | | | |
|---|---|---|---|
| CITRI RETICULATAE PERICARPIUM 94 - 156 | PINELLIAE RHIZOMA 94 -156 | ASTERIS RADIX ET RHIZOMA 94 - 156 | FARFARAE FLOS 94 - 156 |
| PORIA 94 - 156 | ATRACTYLODIS MACROCEPHALAE RHIZOMA 94 - 156 | ARMENIACAE SEMEN AMARUM 78 - 130 | PERILLAE FRUCTUS 78 -130 |
| SINAPIS SEMEN 78 - 130 | RAPHANI SEMEN 78 - 130 | CINNAMOMI RAMULUS 46-78 | SCUTELLARIAE RADIX 94 - 156 |
| FORSYTHIAE FRUCTUS 94 - 156 | HOUTTUYNIAE HERBA 225 - 391 | et GLYCYRRHIZAE RADIX ET RHIZOMA 31 - 53. | |

2. Composition pharmaceutique pour une utilisation dans le traitement de la bronchite selon la revendication 1, **caractérisée en ce que** ladite composition pharmaceutique est préparée à partir des produits à visée médicale dans les parties en poids suivantes :
| | | | |
|---|---|---|---|
| CITRI RETICULATAE PERICARPIUM 94 | PINELLIAE RHIZOMA 156 | ASTERIS RADIX ET RHIZOMA 94 | FARFARAE FLOS 156 |
| PORIA 94 | ATRACTYLODIS MACROCEPHALAE RHIZOMA 156 | ARMENIACAE SEMEN AMARUM 78 | PERILLAE FRUCTUS 130 |
| SINAPIS SEMEN 78 | RAPHANI SEMEN 130 | CINNAMOMI RAMULUS 46 | SCUTELLARIAE RADIX 94 |
| FORSYTHIAE FRUCTUS 156 | HOUTTUYNIAE HERBA 391 | et GLYCYRRHIZAE RADIX ET RHIZOMA 31. | |

3. Composition pharmaceutique pour une utilisation dans le traitement de la bronchite selon la revendication 1, **caractérisée en ce que** ladite composition pharmaceutique est préparée à partir des produits à visée médicale dans les parties en poids suivantes :
| | | | |
|---|---|---|---|
| CITRI RETICULATAE PERICARPIUM 156 | PINELLIAE RHIZOMA 94 | ASTERIS RADIX ET RHIZOMA 156 | FARFARAE FLOS 94 |
| PORIA 156 | ATRACTYLODIS MACROCEPHALAE RHIZOMA 94 | ARMENIACAE SEMEN AMARUM 130 | PERILLAE FRUCTUS 78 |
| SINAPIS SEMEN 130 | RAPHANI SEMEN 78 | CINNAMOMI RAMULUS 78 | SCUTELLARIAE RADIX 156 |
| FORSYTHIAE FRUCTUS 94 | HOUTTUYNIAE HERBA 225 | et GLYCYRRHIZAE RADIX ET RHIZOMA 53. | |

4. Composition pharmaceutique pour une utilisation dans le traitement de la bronchite selon la revendication 1, **caractérisée en ce que** ladite composition pharmaceutique est préparée à partir des produits à visée médicale dans les parties en poids suivantes :
| | | | |
|---|---|---|---|
| CITRI RETICULATAE PERICARPIUM 125 | PINELLIAE RHIZOMA 125 | ASTERIS RADIX ET RHIZOMA 125 | FARFARAE FLOS 125 |
| PORIA 125 | ATRACTYLODIS MACROCEPHALAE RHIZOMA 125 | ARMENIACAE SEMEN AMARUM 104 | PERILLAE FRUCTUS 104 |
| SINAPIS SEMEN 104 | RAPHANI SEMEN 104 | CINNAMOMI RAMULUS 62 | SCUTELLARIAE RADIX 125 |
| FORSYTHIAE FRUCTUS 125 | HOUTTUYNIAE HERBA 313 | et GLYCYRRHIZAE RADIX ET RHIZOMA 42. | |

5. Composition pharmaceutique pour une utilisation dans le traitement de la bronchite selon la revendication 1, **caractérisée en ce que** ladite composition pharmaceutique est préparée à partir des produits à visée médicale dans les parties en poids suivantes :
| | | | |
|---|---|---|---|
| CITRI RETICULATAE PERICARPIUM 98 | PINELLIAE RHIZOMA 98 | ASTERIS RADIX ET RHIZOMA 104 | FARFARAE FLOS 104 |
| PORIA 144 | ATRACTYLODIS MACROCEPHALAE RHIZOMA 148 | ARMENIACAE SEMEN AMARUM 85 | PERILLAE FRUCTUS 85 |
| SINAPIS SEMEN 125 | RAPHANI SEMEN 128 | CINNAMOMI RAMULUS 75 | SCUTELLARIAE RADIX 151 |
| FORSYTHIAE FRUCTUS 149 | HOUTTUYNIAE HERBA 377 | et GLYCYRRHIZAE RADIX ET RHIZOMA 49. | |

6. Composition pharmaceutique pour une utilisation dans le traitement de la bronchite selon la revendication 1, **caractérisée en ce que** ladite composition pharmaceutique est préparée à partir des produits à visée médicale dans les parties en poids suivantes :
| | | | |
|---|---|---|---|
| CITRI RETICULATAE PERICARPIUM 147 | PINELLIAE RHIZOMA 152 | ASTERIS RADIX ET RHIZOMA 149 | FARFARAE FLOS 140 |
| PORIA 105 | ATRACTYLODIS MACROCEPHALAE RHIZOMA 105 | ARMENIACAE SEMEN AMARUM 81 | PERILLAE FRUCTUS 81 |
| SINAPIS SEMEN 125 | RAPHANI SEMEN 125 | CINNAMOMI RAMULUS 74 | SCUTELLARIAE RADIX 149 |
| FORSYTHIAE FRUCTUS 105 | HOUTTUYNIAE HERBA 276 | et GLYCYRRHIZAE RADIX ET RHIZOMA 45. | |

7. Composition pharmaceutique pour une utilisation selon l'une quelconque des revendications 1 à 6, **caractérisée en ce que** le PINELLIAE RHIZOMA est du PINELLIAE RHIZOMA PRAEPARATUM CUM ALUMINE, l'ATRACTYLODIS MACROCEPHALAE RHIZOMA est du RHIZOMA ATRACTYLODIS MACROCEPHALAE TOSTUM CUM MELLE ET FURFURE, l'ARMENIACAE SEMEN AMARUM est de l'ARMENIACAE SEMEN AMARUM TOSTUM, et le SCUTELLARIAE RADIX se présente sous la forme de tranches.

8. Composition pharmaceutique pour une utilisation selon l'une quelconque des revendications 1 à 7, **caractérisée en ce que** la composition pharmaceutique est préparée en suivant les étapes consistant à :
(1) peser du SCUTELLARIAE RADIX propre sélectionné conformément à la partie exposée dans la prescription et mettre celui-ci sous la forme d'une poudre fine en vue d'une utilisation ultérieure ;
(2) peser du CITRI RETICULATAE PERICARPIUM et du CINNAMOMI RAMULUS propres sélectionnés conformément aux parties exposées dans la prescription, en ajoutant 8 à 12 fois en v/p (ml/g) d'eau par rapport au poids des produits à visée médicale, et soumettre le mélange à une distillation pendant 3 à 7 h pour extraire les huiles essentielles, recueillir les huiles essentielles en vue d'une utilisation ultérieure ;
(3) recueillir la solution aqueuse obtenue après la distillation de l'étape (2) dans un récipient, extraire le résidu de plantes médicinales de l'étape (2) conjointement avec l'ATRACTYLODIS MACROCEPHALAE RHIZOMA, le PORIA, le GLYCYRRHIZAE RADIX ET RHIZOMA, le FARFARAE FLOS et le FORSYTHIAE FRUCTUS deux fois avec de l'eau, chaque fois pendant 1 à 3 heures, la quantité d'eau représentant 7 à 11 fois en v/p (ml/g) le poids des produits à visée médicale la première fois et 5 à 9 fois en v/p (ml/g) la deuxième fois ; puis regrouper les extraits aqueux et filtrer le mélange, en combinant le filtrat avec ladite solution aqueuse décrite ci-dessus obtenue après la distillation, et concentrer jusqu'à obtenir une pâte claire d'une densité relative de 1,15 à 1,20 mesurée thermiquement à 60 °C en vue d'une utilisation ultérieure ;
(4) peser du PINELLIAE RHIZOMA, du PERILLAE FRUCTUS, du SINAPIS SEMEN, du RAPHANI SEMEN, de l'ASTERIS RADIX ET RHIZOMA, de l'HOUTTUYNIAE HERBA et de l'ARMENIACAE SEMEN AMARUM propres sélectionnés conformément aux parties exposées dans la prescription, extraire ceux-ci deux fois dans 5 à 7 fois en v/p (ml/g) d'une solution d'éthanol à 40-70 % par rapport au poids des produits à visée médicale, la première fois pendant 1 à 3 heures et la deuxième fois pendant 1 à 2 heures ; regrouper les extraits et filtrer le mélange ; récupérer l'éthanol du filtrat, concentrer le résidu jusqu'à obtenir une pâte claire d'une densité relative de 1,15 à 1,20 mesurée thermiquement à 60 °C, et combiner la pâte claire avec la pâte claire obtenue à l'étape (3) en vue d'une utilisation ultérieure ;
la fine poudre obtenue à l'étape (1), les huiles essentielles obtenue à l'étape (2) et les pâtes claires combinées obtenues à l'étape (4) constituent les substances actives de la composition pharmaceutique.

9. Composition pharmaceutique pour une utilisation selon l'une quelconque des revendications 1 à 8, **caractérisée en ce que** la composition pharmaceutique est sous une forme galénique de capsule dure, de comprimé, de poudre, de liquide oral, de capsule molle, de pilule, de teinture, de sirop, de suppositoire, de gel, de spray ou d'injection.

10. Composition pharmaceutique pour une utilisation selon les revendications 9, **caractérisée en ce qu'**un comprimé de la composition pharmaceutique est formulé en suivant les étapes consistant à :
(1) peser du SCUTELLARIAE RADIX propre sélectionné conformément à la partie exposée dans la prescription et mettre celui-ci sous la forme d'une poudre fine en vue d'une utilisation ultérieure de SCUTELLARIAE RADIX ;
(2) peser du CITRI RETICULATAE PERICARPIUM et du CINNAMOMI RAMULUS propres sélectionnés conformément aux parties exposées dans la prescription, en ajoutant 8 à 12 fois en v/p (ml/g) d'eau par rapport au poids des produits à visée médicale, soumettre le mélange à une distillation pendant 4 à 6 h pour extraire les huiles essentielles, et recueillir les huiles essentielles en vue d'une utilisation ultérieure ;
(3) recueillir la solution aqueuse obtenue après la distillation de l'étape (2) dans un récipient, extraire le résidu de plantes médicinales de l'étape (2) conjointement avec l'ATRACTYLODIS MACROCEPHALAE RHIZOMA, le PORIA, le GLYCYRRHIZAE RADIX ET RHIZOMA, le FARFARAE FLOS et le FORSYTHIAE FRUCTUS deux fois avec de l'eau, chaque fois pendant 1 à 3 heures, la quantité d'eau représentant 7 à 11 fois en v/p (ml/g) le poids des produits à visée médicale la première fois et 5 à 9 fois en v/p (ml/g) la deuxième fois ; puis regrouper les extraits aqueux et filtrer le mélange, en combinant le filtrat avec ladite solution aqueuse décrite ci-dessus obtenue après la distillation, et concentrer jusqu'à obtenir une pâte claire d'une densité relative de 1,15 à 1,20 mesurée thermiquement à 60 °C en vue d'une utilisation ultérieure ;
(4) peser du PINELLIAE RHIZOMA, du PERILLAE FRUCTUS, du SINAPIS SEMEN, du RAPHANI SEMEN, de l'ASTERIS RADIX ET RHIZOMA, de l'HOUTTUYNIAE HERBA et de l'ARMENIACAE SEMEN AMARUM propres sélectionnés conformément aux parties exposées dans la prescription, extraire ceux-ci deux fois avec 5 à 7 fois en v/p (ml/g) d'une solution d'éthanol à 40-70 % par rapport au poids des produits à visée médicale, la première fois pendant 1 à 3 heures et la deuxième fois pendant 1 à 2 heures ; regrouper les extraits et filtrer le mélange ; récupérer l'éthanol du filtrat, concentrer le résidu jusqu'à obtenir une pâte claire d'une densité relative de 1,15 à 1,20 mesurée thermiquement à 60 °C, et combiner la pâte claire avec la pâte claire obtenue à l'étape (3) en vue d'une utilisation ultérieure ;
(5) mettre sous forme de granulés les pâtes claires combinées obtenues à l'étape (4) avec la fine poudre obtenue à l'étape (1) en tant que produit de base, et finir les granulés obtenus en vue d'une utilisation ultérieure ;
(6) fournir les produits en parties en poids en vue de la fabrication des comprimés comme suit :
granulés obtenus à l'étape (5) 360 - 600 dioxyde de silicium colloïdal 1,8 - 3,2 stéarate de magnésium 1,8 - 3,2
(7) fabriquer les comprimés par un procédé de formulation conventionnel pour obtenir le comprimé de la composition pharmaceutique.

11. Composition pharmaceutique pour une utilisation selon la revendication 10, **caractérisée en ce que** le comprimé de la composition pharmaceutique est formulé en suivant les étapes consistant à :
(1) peser du SCUTELLARIAE RADIX propre sélectionné conformément à la partie exposée dans la prescription et mettre celui-ci sous la forme d'une poudre fine en vue d'une utilisation ultérieure de SCUTELLARIAE RADIX ;
(2) peser du CITRI RETICULATAE PERICARPIUM et du CINNAMOMI RAMULUS propres sélectionnés conformément aux parties exposées dans la prescription, ajouter 9 fois en v/p (ml/g) d'eau par rapport au poids des produits à visée médicale, soumettre le mélange à une distillation pendant 5 h pour extraire les huiles essentielles, et recueillir les huiles essentielles en vue d'une utilisation ultérieure ;
(3) recueillir la solution aqueuse obtenue après la distillation de l'étape (2) dans un récipient, extraire le résidu de plantes médicinales de l'étape (2) conjointement avec l'ATRACTYLODIS MACROCEPHALAE RHIZOMA, le PORIA, le GLYCYRRHIZAE RADIX ET RHIZOMA, le FARFARAE FLOS et le FORSYTHIAE FRUCTUS deux fois avec de l'eau, chaque fois pendant 4 heures, la quantité d'eau représentant 10 fois en v/p (ml/g) le poids des produits à visée médicale la première fois et 7 fois en v/p (ml/g) la deuxième fois ; puis regrouper les extraits aqueux et filtrer le mélange, en combinant le filtrat avec ladite solution aqueuse décrite ci-dessus obtenue après la distillation, et concentrer jusqu'à obtenir une pâte claire d'une densité relative de 1,17 mesurée thermiquement à 60 °C en vue d'une utilisation ultérieure ;
(4) peser du PINELLIAE RHIZOMA, du PERILLAE FRUCTUS, du SINAPIS SEMEN, du RAPHANI SEMEN, de l'ASTERIS RADIX ET RHIZOMA, de l'HOUTTUYNIAE HERBA et de l'ARMENIACAE SEMEN AMARUM propres sélectionnés conformément aux parties exposées dans la prescription, extraire ceux-ci deux fois avec 6 fois en v/p (ml/g) d'une solution d'éthanol à 60 % par rapport au poids des produits à visée médicale, la première fois pendant 2 heures et la deuxième fois pendant 1 heure ; regrouper les extraits et filtrer le mélange ; récupérer l'éthanol du filtrat, concentrer le résidu jusqu'à obtenir une pâte claire d'une densité relative de 1,17 mesurée thermiquement à 60 °C, et combiner la pâte claire avec la pâte claire obtenue à l'étape (3) en vue d'une utilisation ultérieure ;
(5) mettre sous forme de granulés les pâtes claires combinées obtenues à l'étape (4) avec la fine poudre obtenue à l'étape (1) en tant que produit de base, et finir les granulés obtenus en vue d'une utilisation ultérieure,
(6) fournir les produits en parties en poids en vue de la fabrication des comprimés comme suit :
granulés obtenus à l'étape (5) 470 dioxyde de silicium colloïdal 2,4
stéarate de magnésium 2,4 ;
(7) ajouter les huiles essentielles obtenues à l'étape (2) au dioxyde de silicium colloïdal et fabriquer les comprimés par un procédé de formulation conventionnel pour obtenir le comprimé de la composition pharmaceutique.

12. Composition pharmaceutique pour une utilisation dans le traitement de la bronchite selon l'une quelconque des revendications 1 à 11, **caractérisée en ce que** ladite bronchite est une bronchite chronique.
